# EUROPÄISCHE PATENTANMELDUNG

(11) **EP 2 336 103 A2**
(43) Veröffentlichungstag der Anmeldung: **22.06.2011**
(21) Anmeldenummer: 10014791.7
(22) Anmeldetag: 20.07.2006
(51) Int. Cl.: C07C 215/64, A61K 31/135

(54) **HCL-Polymorphe von 3-[2-(Dimethylamino)methyl-(cyclohex-1-yl)]phenol**

(30) Priorität: 22.07.2005 DE 102005034974; 22.07.2005 DE 102005034973
(62) Teilanmeldung aus: 06776327.6
(71) Anmelder: Grünenthal GmbH, 52078 Aachen (DE)
(72) Erfinder: Gruss, Michael, Dr., 52062 Aachen (DE); Fischer, Andreas, Dr., 52393 Hürtgenwald (DE); Kegel, Markus, Dr., 41239 Mönchengladbach (DE); Hell, Wolfgang, Dr., 52066 Aachen (DE); Von Raumer, Markus, 4144 Arlesheim (CH); Berghausen, Jörg, 79541 Lörrach (DE); De Paul, Susan, Margaret, 8057 Zürich (CH)
(74) Vertreter: Kutzenberger, Helga

(57) **Zusammenfassung**

Die vorliegende Erfindung betrifft ein kristallines Salz von 3-[2-(Dimethylamino)methyl-(cyclohex-1-yl)]-phenol und Chlorwasserstoff, bevorzugt in einer Zusammensetzung von 1: 1, verschiedene kristalline Formen dieses Salzes sowie Verfahren zu deren Herstellung, eine pharmazeutische Zusammensetzung und die Verwendung des Salzes als pharmazeutischer Wirkstoff in einem Arzneimittel.

## Beschreibung

Die vorliegende Erfindung betrifft ein kristallines Salz von 3-[2-(Dimethylamino)methyl-(cyclohex-1-yl)]-phenol und Chlorwasserstoff, bevorzugt in einer Zusammensetzung von 1:1, verschiedene kristalline Formen dieses Salzes sowie Verfahren zu deren Herstellung, eine pharmazeutische Zusammensetzung und die Verwendung des Salzes als pharmazeutischer Wirkstoff in einem Arzneimittel.

In der EP-A1-0 753 506 werden 3-[2-(Dimethylamino)methyl-(cyclohex-1-yl)]-phenole mit analgetischer Wirkung beschrieben. In der Beschreibung wird erwähnt, dass man auch Salze aus den freien Basen dieser Verbindungen herstellen kann, wie beispielsweise Hydrochloride. Die EP-A1-0 753 506 enthält aber keinerlei Hinweise darauf, dass diese Hydrochloride als kristalline Festsubstanz erhalten werden können.

Es wurde nun überraschend gefunden, dass 3-[2-(Dimethylamino)methyl-(cyclohex-1-yl)]-phenol mit Chlorwasserstoff ein Additionssalz als kristalline Festsubstanz bildet, bevorzugt in einer Zusammensetzung im Verhältnis 1:1. Es wurde ferner gefunden, dass 3-[2-(Dimethylamino)methyl-(cyclohex-1-yl)]-phenol-HCl als kristalline Festkörper polymorphe Formen bilden, die jeweils gezielt herstellbar sind und zumindest teilweise aufgrund ihrer Stabilität besonders als Wirkstoff zur Formulierung von pharmazeutischen Zusammensetzungen geeignet sind. Beispielsweise ist die kristalline Form II aufgrund ihrer kinetischen Stabilität als Wirkstoff zur Formulierung von pharmazeutischen Zusammensetzungen geeignet. Weiterhin ist die kristalline Form V aufgrund ihrer Stabilität in Gegenwart von Wasser, beispielsweise in Form von Luftfeuchtigkeit, als Wirkstoff zur Formulierung von pharmazeutischen Zusammensetzungen geeignet. Ferner wurden gefunden, dass sich die Wydrochloridsalze durch eine sehr gute Löslichkeit in Wasser auszeichnen.

Ein erster Gegenstand der Erfindung sind daher kristalline Salze von Chlorwasserstoff mit 3-[2-(Dimethylamino)methyl-(cyclohex-1-yl))-phenol, wobei 3-[2-Dimethylamino)methyl-(cyclohex-1-yl)-phenol Hydrochlorid der nachstehenden Formel (1) bevorzugt ist.

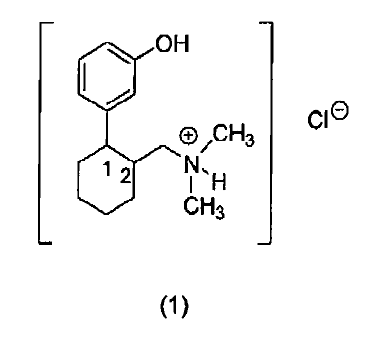

Die Verbindungen der Formel (1) enthalten in den 1- und 2-Positionen des Cyclohexanringes je ein chirales C-Atom. Die Verbindungen der Formel (1) umfassen alle Stereoisomeren und Gemische von Stereoisomeren. Bevorzugt sind Diastereomere oder Gemische von enantiomeren Diastereomeren mit trans-Konfiguration des Phenylringes und der Dimethylaminomethylgruppe (1 R,2R-beziehungsweise 1 S,2S-Konfiguration), wobei das Enantiomer mit der absoluten Konfiguration (1 R,2R) ganz besonders bevorzugt ist.

Die Struktur des (1 R,2R)-Enantiomers von 3-[2-(Dimethylamino)methyl-(cyclohex-1-yl)]-phenol ist nachstehend wiedergegeben:

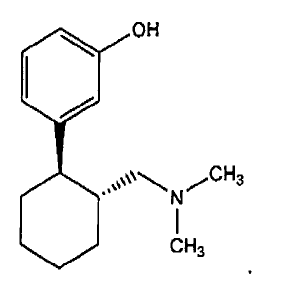

Ein weiterer Gegenstand der Erfindung ist ein Verfahren zur Herstellung von 3-[2-Dimethylamino)methyl-(cyclohex-1-yl)-phenol Hydrochlorid der Formel (1) umfassend die Schritte
a) Lösen oder Suspendieren von 3-[2-(Dimethylamino)methyl-(cyclohex-1-yl)]phenol in einem Lösungsmittel, oder Vorlegen von 3-[2-(Dimethylamino)methyl-(cyclohex-1-yl)]phenol in Substanz,
b) Vermischen der Lösung, des Feststoffs oder der Suspension mit einer Lösung von Chlorwasserstoff, insbesondere Salzsäure, gegebenenfalls Abkühlen oder Erwärmen und Halten bei einer Temperatur zwischen - 80 °C und 150 °C, bevorzugt zwischen -20 °C und 30 °C, besonders bevorzugt zwischen - 5 °C und 5 °C, bis zur vollständigen Bildung eines Feststoffs, und
c) Isolieren der Verbindung der Formel (1 ).

Alternativ kann selbstverständlich auch 3-[2-(Dimethylamino)methyl-(cyclohex-1-yl)]phenol in Lösung, als Suspension oder in Substanz in eine Lösung oder Gas enthaltend Hydrogenchlorid eingetragen werden.

Die Salzsäure in Schritt b) des vorstehen genannten Verfahrens kann insbesondere als eine wässrige Lösung vorliegen oder als eine Lösung in einem organischen Lösungsmittel, bevorzugt in einem Lösungsmittel ausgewählt aus der Gruppe bestehend aus Diethylether, tert-Butylmethylether und Tetrahydrofuran.

Ebenfalls ein Gegenstand der Erfindung ist ein Verfahren zur Herstellung von 3-[2-Dimethylamino)methyl-(cyclohex-1-yl)-phenol Hydrochlorid der Formel (1) umfassend die Schritte
a) Lösen von 3-[2-(Dimethylamino)methyl-(cyclohex-1-yl)]phenol in einem Lösungsmittel, oder Voriegen von 3-[2-(Dimethylamino)methyl-(cyclohex-1-yl)]phenol in Substanz, und
b) Einleiten von Chlorwasserstoffgas in die Lösung oder Suspension bzw. Überleiten von Chlorwasserstoffgas über den Feststoff.

Die Verbindungen der Formel (1) werden beim erfindungsgemäßen Verfahren der Salzbildung auch in einer amorphen Form erhalten. Amorphe Formen der Verbindungen der Formel (1) sind zum Beispiel einfach mittels Gefriertrocknung beziehungsweise schnellem Abkühlen von Lösungen erhältlich. Amorphe Verbindungen der Formel (1) sind nicht sehr stabil und neigen in Gegenwart von Feuchtigkeit zur Bildung von Hydraten. Ebenfalls eignen sich amorphe Formen der Verbindungen der Formel (1) in solvatisierenden Lösungsmitteln wie beispielsweise Aceton, Ethanol, Methanol, Methylethylketon, Isopropanol, n-Propanol und n-Octanol zur Herstellung der entsprechenden Solvate. Die amorphe Form der Verbindungen der Formel (1) lässt sich ebenfalls zur gezielten Herstellung von kristallinen Formen nutzen.

Es wurde gefunden, dass die Verbindungen der Formel (1) als kristalline Festkörper polymorphe Formen bilden, die gezielt aus 3-[2-Dimethylamino)methyl-(cyclohex-1-yl)-phenol Hydrochlorid herstellbar sind und aufgrund ihrer Stabilität besonders als Wirkstoff zur Formulierung von pharmazeutischen Zusammensetzungen geeignet sind. Bevorzugt ist die kristalline Form II aufgrund ihrer kinetischen Stabilität als Wirkstoff zur Formulierung von pharmazeutischen Zusammensetzungen geeignet. Ebenfalls bevorzugt ist die kristalline Form V aufgrund ihrer Stabilität in Gegenwart von Wasser, beispielsweise in Form von Luftfeuchtigkeit, als Wirkstoff zur Formulierung von pharmazeutischen Zusammensetzungen geeignet.

Es ist bekannt, unter anderem aus Z. Jane Li et al. in J. Pharm. Sci., 1999, Vol. 88(3), Seiten 337 bis 346, dass Enantiomere identische Röntgen-Diffraktogramme und Raman-Spektren ergeben und somit gleiche polymorphe Formen bilden. In Rahmen dieser Erfindung werden somit polymorphe Formen von allen Enantiomeren umfaßt.

Ein weiterer Gegenstand der Erfindung ist eine kristalline Form von 3-[2-Dimethylamino)methyl-(cyclohex-1-yl)-phenol Hydrochlorid der Formel (1), die ein charakteristisches Röntgen-Beugungsbild im Bereich von 2° bis 35° 2θ mit ausgeprägten charakteristischen Signalen (Peaks) aufweist, ausgedrückt in 2 Theta-Werten:
11.2 (w), 14.1 (m), 17.1 (w), 19.5 (w), 19.8 (vs), 20.5 (w), 21.5 (m), 24.1 (m), 26.1 (s), 26.8 (w), 31.3 (m);

### nachfolgend als Form I bezeichnet.

Ein weiterer Gegenstand der Erfindung ist eine kristalline Form I von 3-[2-(Dimethylamino)methyl-(cyclohex-1-yl)]-phenol-HCl der Formel I, die ein charakteristisches Röntgen-Beugungsbild mit den nachstehenden ausgeprägten Reflexen aufweist.

Die Meßgenauigkeit der 2Theta-Werte liegt im Bereich von ± 0,2.

| 2Theta | Intensität (relativ) |
|---|---|
| 9,09 | 1 |
| 10,22 | 1 |
| 11,22 | 24 |
| 12,21 | 4 |
| 13,03 | 2 |
| 13,47 | 3 |
| 14,12 | 43 |
| 14,90 | 15 |
| 16,69 | 3 |
| 17,16 | 24 |
| 18,05 | 10 |
| 18,89 | 14 |
| 19,53 | 26 |
| 19,78 | 100 |
| 20,22 | 12 |
| 20,48 | 28 |
| 21,46 | 43 |
| 22,24 | 11 |
| 22,50 | 21 |
| 22,71 | 19 |
| 24,12 | 34 |
| 26,09 | 73 |
| 26,81 | 43 |
| 27,68 | 22 |
| 28,26 | 25 |
| 28,51 | 14 |
| 29,96 | 14 |
| 31,28 | 46 |
| 32,58 | 8 |
| 33,03 | 6 |
| 34,52 | 15 |
| 35,44 | 28 |
| 35,67 | 15 |
| 36,38 | 8 |
| 37,94 | 8 |
| 38,59 | 8 |
| 39,66 | 18 |
| 40,13 | 15 |
| 40,88 | 14 |
| 41,72 | 21 |
| 42,44 | 9 |
| 43,15 | 44 |
| 44,35 | 47 |
| 45,70 | 10 |
| 46,55 | 11 |
| 47,13 | 8 |
| 47,84 | 15 |
| 48,69 | 10 |
| 49,68 | 14 |

Zuvor und nachfolgend bedeuten die Abkürzungen in Klammern: (vs) = sehr starke Intensität, (s) = starke Intensität, (m) = mittlere Intensität, (w) = schwache Intensität und (vw) = sehr schwache Intensität. Die Abkürzung "sh" in den Tabellen der Raman-Spektren bedeutet Schulter.

Die kristalline Form I von 3-[2-Dimethylamino)methyl-(cyclohex-1-yl)-phenol Hydrochlorid der Formel (1) weist ein charakteristisches Raman-Spektrum mit charakteristischen Banden auf, das durch die folgenden Wellenzahlen (cm⁻¹) beschrieben wird:

| Wellenzahl cm⁻¹ | Intensität |
|---|---|
| 82 | S |
| 107 | VS |
| 177 | W |
| 251 | M |
| 284 | VW |
| 346 | W |
| 362 | VW |

| Wellenzahl [cm⁻¹] | Intensität |
|---|---|
| 423 | VW |
| 434 | VW |
| 479 | VW |
| 533 | W |
| 626 | \/W |
| 634 | VW |
| 754 | VW |
| 762 | W |
| 787 | VW |
| 794 | VW |
| 823 | W |
| 838 | W |
| 861 | VW |
| 884 | VW |
| 936 | VW |
| 954 | VW |
| 972 | VW |
| 1001 | VS |
| 1056 | W |
| 1070 | W |
| 1080 | VW |
| 1102 | VW |
| 1122 | VW |
| 1154 | VW |
| 1162 | VW |
| 1184 | VW |
| 1208 | VW |
| 1227 | VW |
| 1274 | W |
| 1286 | W |
| 1293 | W |
| 1306 | W |
| 1316 | W |
| 1335 | VW |
| 1350 | VW |
| 1367 | VW |
| 1439 | W |
| 1449 | W |
| 1470 | W |
| 1584 | W |
| 1611 | W |
| 2633 | VW |
| 2662 | VW |
| 2714 | VW |
| 2805 | VW |
| 2856 | M |
| 2901 | M |
| 2922 | M |
| 2942 | M |
| 2957 | M |
| 2983 | W |
| 3008 | W |
| 3017 | W |
| 3025 | W |
| 3042 | W |
| 3051 | M |
| 3074 | VW |
| 3196 | VW |

Die Angabe der Wellenzahlen erfolgt mit einer Genauigkeit von ± 4 cm⁻¹

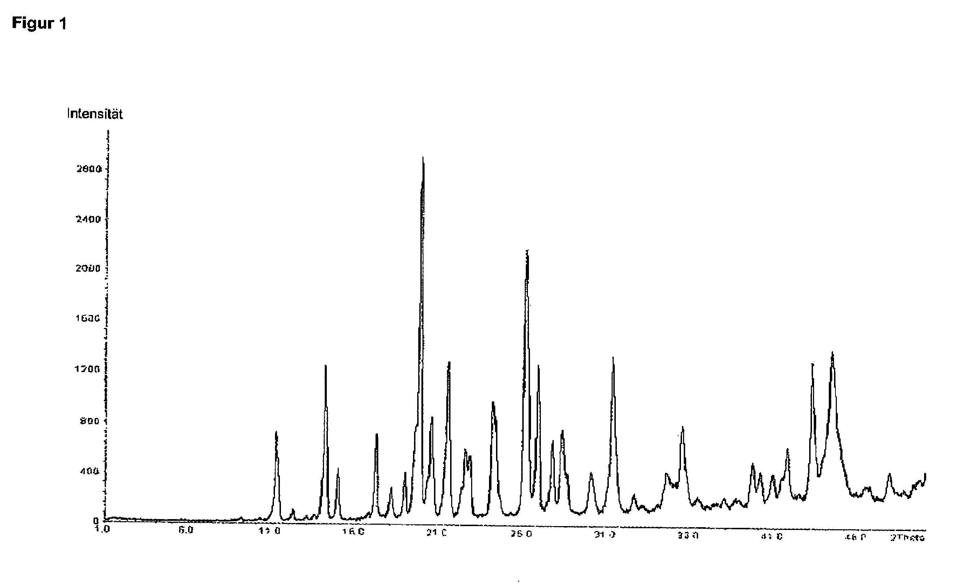

Ein weiterer Gegenstand der Erfindung ist eine kristalline Form I von 3-[2-Dimethylamino)methyl-(cyclohex-1-yl)-phenol Hydrochlorid der Formel (1), die ein charakteristisches Röntgen-Beugungsbild, wie in Figur 1 dargestellt besitzt.

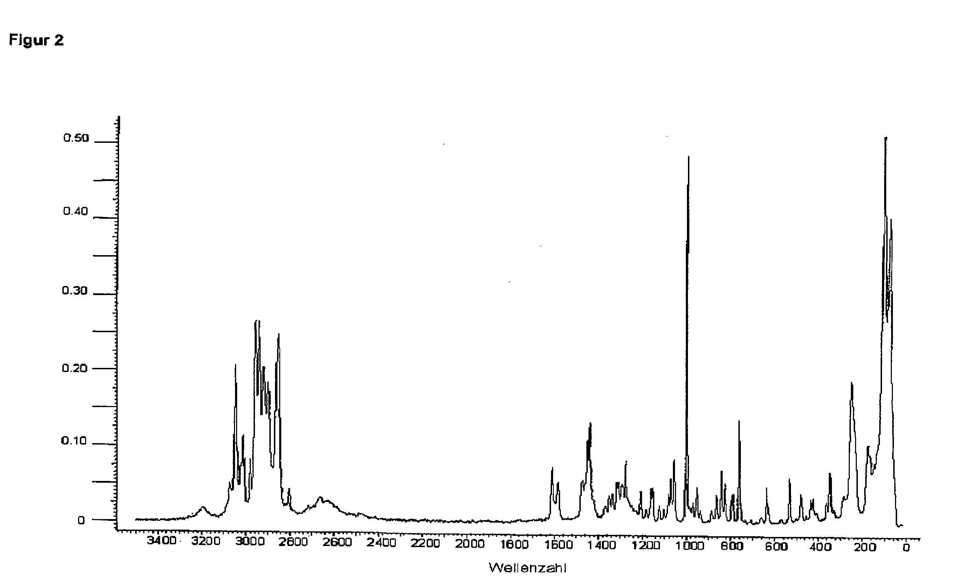

Ein weiterer Gegenstand der Erfindung ist eine kristalline Form I von 3-[2-Dimethylamino)methyl-(cyclohex-1-yl)phenol Hydrochlorid der Formel (1), die durch ein Raman-Spektrum wie in Figur 2 dargestellt charakterisiert wird.

Die kristalline Form I list die thermodynamisch stabilste Form bei niedrigen Temperaturen bis etwa 40 °C. Die kristalline Form I bildet in Anwesenheit von Luftfeuchtigkeit bei einer relativen Luftfeuchtigkeit > 50% irreversibel Hydrate der kristallinen Form V aus. Bei einer relativen Luftfeuchtigkeit > 95 % ergeben sich Hydrate mit einem Anteil von Kristallwasser bezogen auf das Gesamtgewicht des Hydrates im Bereich von 8 % bis 10%. Um die Bildung von Hydraten zu vermeiden, können die Verbindungen der kristallinen Form I vorteilhafterweise in einer feuchtigkeitsarmen Umgebung beispielsweise in einem Gefäß über Phosphorpentoxid oder Molekularsieb gelagert werden. Ebenfalls ist eine Lagerung der kristallinen Form I unter trockenem Schutzgas (zum Beispiel Stickstoff) empfehlenswert. Der Schmelzpunkt beträgt etwa 150 °C.

Polymorph I kann als festes Pulver mit gewünschten mittleren Partikelgrößen hergestellt werden, die in der Regel im Bereich von 1 µm bis etwa 500 µm liegen.

Die Verbindung der Formel (1) bildet eine weitere, bei höheren Temperaturen thermodynamisch stabile kristalline Form II, die bei Normalbedingungen an Luft und unter Ausschluß von Luftfeuchtigkeit ebenfalls stabil ist. Die kristalline Form II ist ebenfalls so handhabbar, dass sie für die Herstellung pharmazeutischer Zusammensetzungen eingesetzt werden kann.

Ein weiterer Gegenstand der Erfindung ist eine kristalline Form von 3-[2-Dimethylamino)methyl-(cyclohex-1-yl)-phenol Hydrochlorid der Formel (1), die ein charakteristisches Röntgen-Beugungsbild im Bereich von 2° bis 35° 2θ mit ausgeprägten charakteristischen Linien aufweist, ausgedrückt in 2Theta-Werte:
11.1 (m), 12.9 (w), 16.1 (m), 17.1 (w), 19.1 (s), 19.6 (w), 19.9 (m), 23.2 (w), 25.8 (W), 26.1 (s), 33.6 (w);
nachfolgend als Form II bezeichnet.

Ein weiterer Gegenstand der Erfindung ist eine kristalline Form II von 3-[2-(Dimethylamino)methyl-(cyclohex-1-yl)]-phenol-HCl der Formel I, die ein charakteristisches Röntgen-Beugungsbild mit den nachstehenden ausgeprägten Reflexen aufweist.

| 2Theta | Intensität(relativ) |
|---|---|
| 11,06 | 56 |
| 11,55 | 12 |
| 12,97 | 27 |
| 13,70 | 18 |
| 14,10 | 6 |
| 15,03 | 9 |
| 16,15 | 55 |
| 17,07 | 25 |
| 18.48 | 4 |
| 19,10 | 100 |
| 19,56 | 25 |
| 19,90 | 36 |
| 21,13 | 11 |
| 21,95 | 9 |
| 22,21 | 4 |
| 22,66 | 15 |
| 23,26 | 26 |
| 24,64 | 15 |
| 24,95 | 4 |
| 25,43 | 7 |
| 25,82 | 22 |
| 26,12 | 82 |
| 26,71 | 4 |
| 27,02 | 19 |
| 27,66 | 8 |
| 28,44 | 6 |
| 28,72 | 5 |
| 29,07 | 3 |
| 29,65 | 7 |
| 30,34 | 6 |
| 31,44 | 5 |
| 31,95 | 6 |
| 32,42 | 14 |
| 33,62 | 15 |
| 33,99 | 7 |
| 34,64 | 7 |
| 35,21 | 5 |
| 36,17 | 10 |
| 37,39 | 2 |
| 38,30 | 3 |
| 38,96 | 3 |
| 39,24 | 4 |
| 39,62 | 10 |
| 40,35 | 2 |
| 41,26 | 4 |
| 41,82 | 3 |
| 42,38 | 3 |
| 42,89 | 5 |
| 44,16 | 3 |
| 44,76 | 4 |
| 45,35 | 11 |
| 45,85 | 4 |
| 46,18 | 2 |
| 47,09 | 3 |
| 48,45 | 3 |

Ein weiterer Gegenstand der Erfindung ist eine kristalline Form II von 3-[2-Dimethylamino)methyl-(cyclohex-1-yl)-phenol Hydrochlorid der Formel (1), die ein charakteristisches Raman-Spektrum mit charakteristischen Banden aufweist, das durch die folgenden Wellenzahlen (cm⁻¹) beschrieben wird

| Wellenzahl [cm⁻¹] | Intensität |
|---|---|
| 90 | S |
| 123 | VS |
| 171 | W |
| 230 | VW |
| 248 | M |
| 341 | W |
| 384 | VW |
| 457 | VW |
| 475 | VW |
| 505 | VW |
| 532 | W |
| 573 | VW |
| 624 | VW |
| 638 | W |
| 682 | VW |
| 706 | VW |
| 761 | W |
| 795 | W |
| 817 | VW |
| 844 | W |
| 857 | VW |
| 894 | VW |
| 936 | VW |
| 957 | VW |
| 971 | VW |
| 989 | VW |
| 997 | S |
| 010 | VW |
| 1054 | W |
| 1075 | W |
| 1085 | VW |
| 1124 | VW |
| 1168 | VW |
| 1212 | VW |
| 1250 | VW |
| 1276 | W |
| 1294 | W |
| 1317 | VW |
| 1339 | VW |
| 1355 | VW |
| 1361 | W |
| 1391 | VW |
| 1414 | VW |
| 1441 | W |
| 1462 | W |
| 1529 | VW |
| 1586 | W |
| 1614 | W |
| 2477 | VW |
| 2519 | VW |
| 2665 | VW |
| 2734 | VW |
| 2768 | VW |
| 2814 | VW |
| 2850 | M |
| 2889 | M |
| 2920 | M |
| 2929 | S |
| 2938 | S |
| 2968 | M |
| 3019 | M |
| 3040 | W |
| 3067 | W |
| 3103 | VW |
| 3181 | VW |
| 3229 | VW |
| 3248 | VW |

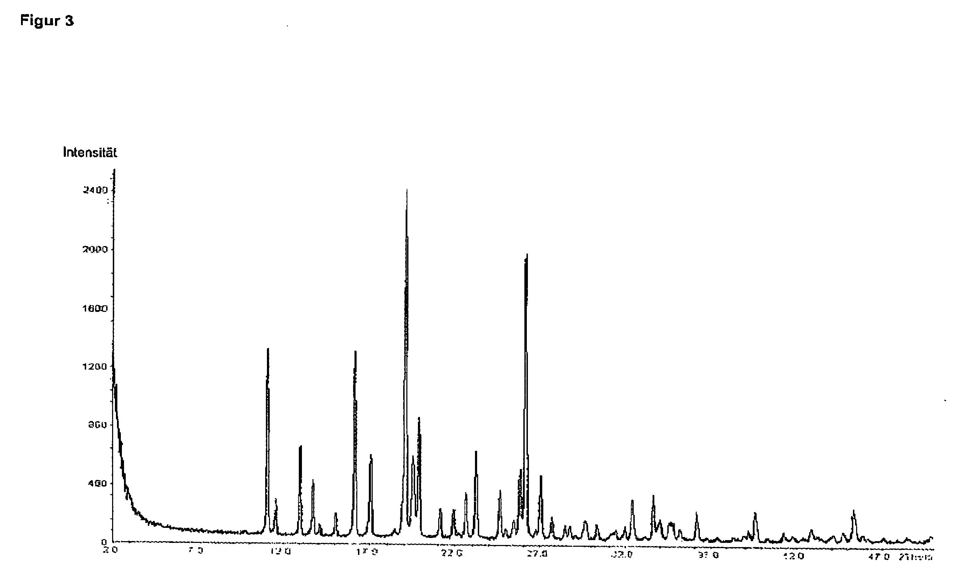

Ein weiterer Gegenstand der Erfindung ist eine kristalline Form II von 3-[2-Dimethylamino)methyl-(cyclohex-1-yl)-phenol Hydrochlorid der Formel (1), die ein charakteristisches Röntgen-Beugungsbild wie in Figur 3 dargestellt besitzt.

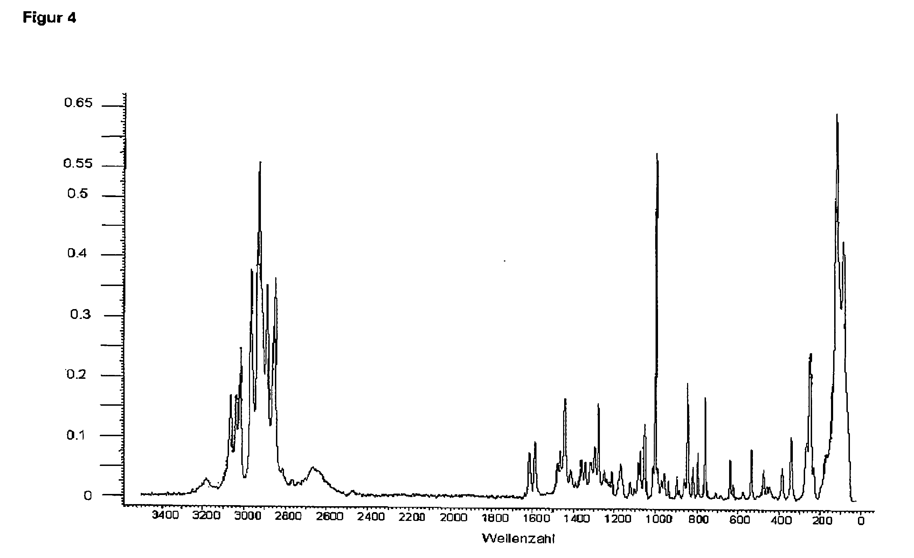

Ein weiterer Gegenstand der Erfindung ist eine kristalline Form II von 3-[2-Dimethylamino)methyl-(cyclohex-1-yl-phenol Hydrochlorid der Formel (1), die durch ein Raman-Spektrum wie in Figur 4 dargestellt charakterisiert wird.

Die Verbindungen der kristallinen Form II sind weniger hygroskopisch als die Verbindungen der kristallinen Formen I, III bzw. IV und bilden erst bei einer relativen Luftfeuchtigkeit > 70 % Hydrate der kristallinen Form V. So ist beispielsweise nach 5 Stunden Lagerung der kristallinen Form II bei einer relativen Luftfeuchtigkeit von 60 % keine nennenswerte Wasseraufnahme festzustellen (Anteil Wasser < 0.15 Gew.-%). Die kristalline Form II wandelt sich mittels Rührmischen in Ethylacetat bei einer Temperatur zwischen 20 °C und 30 °C, bevorzugt bei einer Temperatur von 23 °C, nur langsam In die bel dieser Temperatur thermodynamisch stabilere Form I um, so dass sich erst nach 32 Tagen die kristalline Form II vollständig in die kristalline Form I umgewandelt hat. Ein geringer Anteil der kristallinen Form I lässt sich nach drei Tagen Rührmischen detektieren. Die Umwandlung der kristallinen Form II in die kristalline Form I mittels Rührmischen erfolgt auch in der Anwesenheit von Impfkristallen der kristallinen Form I nur langsam. Die geringe Hygroskopizität und die kinetische Stabilität machen die kristalline Form II zu einem geeigneten Wirkstoff in pharmazeutischen Formulierungen.

Die Verbindungen der kristallinen Form II besitzen eine gute chemische Stabilität. Sie bilden in Anwesenheit von Luftfeuchtigkeit bei einer relativen Luftfeuchtigkeit > 70 °C nur langsam Hydrate der kristallinen Form V aus. Bei einer relativen Luftfeuchtigkeit > 95 % ergeben sich Hydrate mit einem Anteil von Kristallwasser bezogen auf das Gesamtgewicht des Hydrates im Bereich von 8 % bis 10%. Um die Bildung von Hydraten zu vermeiden, werden die Verbindungen der kristallinen Form II vorteilhafterweise in einer feuchtigkeitsarmen Umgebung beispielsweise in einem Gefäß über Phosphorpentoxid oder Molekularsieb gelagert. Ebenfalls ist eine Lagerung der kristallinen Form II unter trockenem Schutzgas (zum Beispiel Stickstoff) empfehlenswert.

Der Schmelzpunkt liegt im Bereich zwischen 175°C und 178 °C und die Schmelzenthalpie beträgt etwa 93.3 J/g, bestimmt mittels DSC bei einer Aufheizrate von 10°C/Minute. Polymorph II kann als festes Pulver mit gewünschten mittleren Partikelgrößen hergestellt werden, die in der Regel im Bereich von 1 µm bis etwa 500 µm liegen.

Die Verbindung der Formel (1) bildet eine weitere stabile kristalline Form III, die bei höheren Temperaturen, bevorzugt bei einer Temperatur zwischen 70 °C und 155 °C, besonders bevorzugt bei einer Temperatur zwischen 75 °C und 151°C, thermodynamisch stabil ist und ebenfalls bei Normalbedingungen an Luft und unter Ausschluß von Luftfeuchtigkeit stabil ist.

Ein weiterer Gegenstand der Erfindung ist eine kristalline Form von 3-[2-Dimethylamino)methyl-(cyclohex-1-yl)-phenol Hydrochlorid der Formel (1), die ein charakteristisches Röntgen-Beugungsbild im Bereich von 2° bis 35° 2θ mit ausgeprägten charakteristischen Linien aufweist, ausgedrückt in 2Theta-Werte: 6.9 (s), 13.9 (m), 16.3 (m), 17.7 (w), 20.9 (vs), 22.1 (w), 22.5 (w), 27.8 (w); nachfolgend als Form III bezeichnet.

Ein weiterer Gegenstand der Erfindung ist eine kristalline Form III von 3-[2-(Dimethylamino)methyl-(cyclohex-1-yl)]-phenol-HCl der Formel I, die ein charakteristisches Röntgen-Beugungsbild mit den nachstehenden ausgeprägten Reflexen aufweist.

| 2Theta | Intensität (relativ) |
|---|---|
| 6,94 | 71 |
| 8,85 | 3 |
| 10.93 | 5 |
| 11,41 | 8 |
| 12,18 | 4 |
| 13,88 | 44 |
| 16,26 | 42 |
| 16,67 | 2 |
| 17,70 | 24 |
| 18,34 | 10 |
| 18,79 | 9 |
| 19,18 | 5 |
| 19,63 | 6 |
| 20,07 | 5 |
| 20,88 | 100 |
| 21,41 | 10 |
| 21,60 | 8 |
| 22,04 | 21 |
| 22,53 | 24 |
| 23,03 | 6 |
| 23,59 | 8 |
| 24,02 | 4 |
| 24,73 | 4 |
| 25,48 | 2 |
| 26,04 | 6 |
| 26,49 | 9 |
| 27,54 | 8 |
| 27,84 | 16 |
| 28,85 | 3 |
| 29,23 | 7 |
| 29,99 | 7 |
| 30,92 | 2 |
| 31,23 | 4 |
| 31,65 | 6 |
| 32,04 | 2 |
| 32,47 | 5 |
| 32,84 | 6 |
| 33,95 | 3 |
| 34,47 | 2 |
| 35,11 | 5 |
| 35,83 | 3 |
| 36,36 | 2 |
| 37,07 | 3 |
| 37,90 | 4 |
| 38,67 | 6 |
| 39,22 | 3 |
| 39,81 | 3 |
| 40,49 | 3 |
| 41,24 | 3 |
| 42,45 | 3 |
| 43,67 | 2 |
| 45,90 | 2 |
| 47,19 | 2 |
| 48,67 | 3 |

Ein weiterer Gegenstand der Erfindung ist eine kristalline Form III von 3-[2-Dimethylamino)methyl-(cyclohex-1-yl)-phenol Hydrochlorid der Formel (1), die ein charakteristisches Raman-Spektrum mit charakteristischen Banden aufweist, das durch die folgenden Wellenzahlen (cm⁻¹) beschrieben wird:

| Wellenzahl [cm⁻¹] | Intensität |
|---|---|
| 75.65 | S |
| 94.93 | VS |
| 4.25 | VW |
| 244.39 | M |
| 286.33 | VW |
| 338.40 | W |
| 386.13 | VW |
| 443.99 | VW |
| 456.04 | VW |
| 481.59 | VW |
| 533.66 | W |
| 573.67 | VW |
| 624.30 | VW |
| 635.87 | VW |
| 702.88 | VW |
| 755.91 | W |
| 784.36 | VW |
| 793.52 | VW |
| 797.86 | VW |
| 819.07 | VW |
| 840.29 | W |
| 848.00 | W |
| 852.34 | W |
| 869.21 | VW |
| 897.66 | VW |
| 936.71 | VW |
| 958.89 | W |
| 974.31 | VW |
| 999.87 | VS |
| 1051.45 | W |
| 1071.70 | W |
| 1103.52 | VW |
| 1124.25 | VW |
| 1147.88 | VW |
| 1159.45 | VW |
| 1167.16 | W |
| 1213.44 | VW |
| 1232.25 | VW |
| 1249.60 | VW |
| 1264.55 | WV |
| 1277.08 | VW |
| 1293.48 | W |
| 1307.94 | W |
| 1311.80 | W |
| 1335.42 | VW |
| 1367.24 | VW |
| 1399.06 | VW |
| 1431.84 | VW |
| 1444.86 | W |
| 1466.07 | W |
| 1479.09 | W |
| 1529.71 | VW |
| 1587.08 | W |
| 1605.40 | W |
| 1653.62 | VW |
| 1661.33 | VW |
| 1704.24 | VW |
| 2675.22 | W |
| 2700.29 | VW |
| 2707.04 | VW |
| 2722.95 | VW |
| 2812.14 | W |
| 2852.16 | S |
| 2858.91 | S |
| 2891.21 | M |
| 2912.91 | M |
| 2924.48 | M |
| 2939.42 | S |
| 2949.55 | M |
| 2960.63 | M |
| 3019.45 | M |
| 3048.86 | M |
| 3071.04 | W |
| 3174.69 | VW |
| 3204.10 | VW |
| 3236.41 | VW |

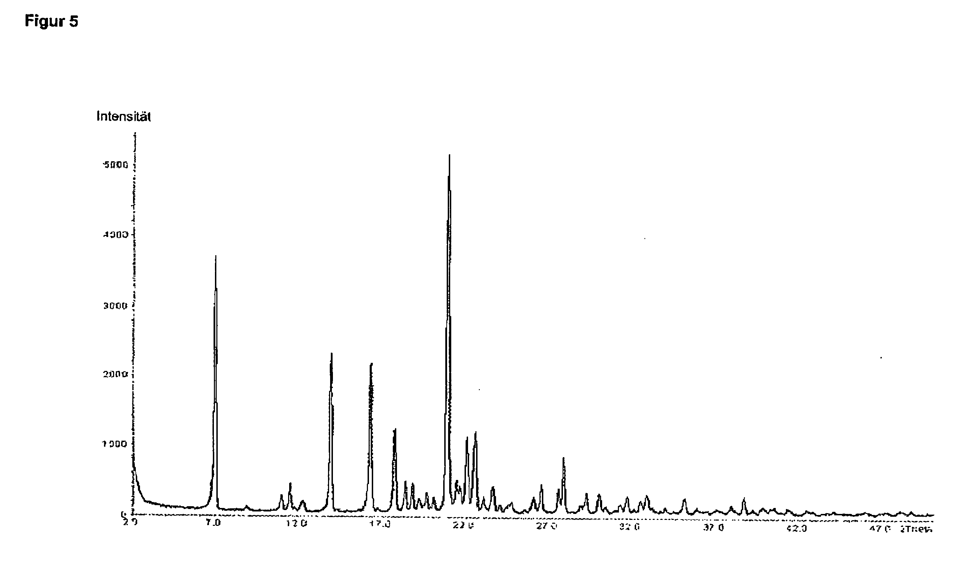

Ein weiterer Gegenstand der Erfindung ist eine kristalline Form III von 3-[2-Dimethylamino)methyl-(cyclohex-1-yl)-phenol Hydrochlorid der Formel (1) die ein charakteristisches Röntgen-Beugungsbild wie in Figur 5 dargestellt besitzt.

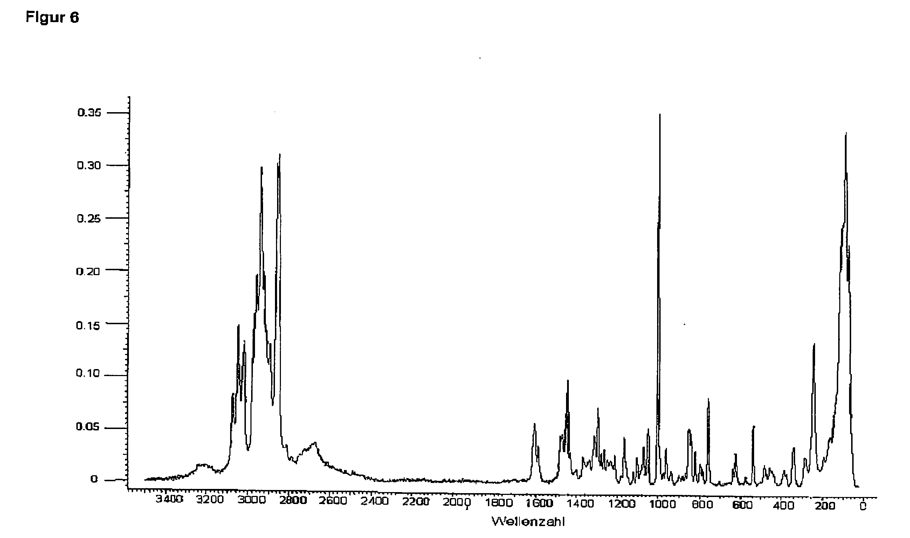

Ein weiterer Gegenstand der Erfindung ist eine kristalline Form III von 3-[2-Dimethylamino)methyl-(cyclohex-1-yl)-phenol Hydrochlorid der Formel (1), die durch ein Raman-Spektrum wie in Figur 6 dargestellt charakterisiert wird.

Die kristalline Form III bildet in von Luftfeuchtigkeit bei einer relativen Luftfeuchtigkeit > 50% irreversibel Hydrate der kristallinen Form V aus. Bei einer relativen Luftfeuchtigkeit > 95 % ergeben sich Hydrate mit einem Anteil von Kristallwasser bezogen auf das Gesamtgewicht des Hydrates im Bereich von 8 % bis 10%. Um die Bildung von Hydraten zu vermeiden, können die Verbindungen der kristallinen Form III vorteilhafterweise in einer feuchtigkeitsarmen Umgebung beispielsweise in einem Gefäß über Phosphorpentoxid oder Molekularsieb gelagert werden. Ebenfalls ist eine Lagerung der kristallinen Form III unter trockenem Schutzgas (zum Beispiel Stickstoff) empfehlenswert.

Der Schmelzpunkt liegt im Bereich zwischen 155 °C und 158 °C und die Schmelzenthalpie beträgt etwa 87 J/g, bestimmt mittels DSC bei einer Aufheizrate von 10°C/Minute. Polymorph III kann als festes Pulver mit gewünschten mittleren Partikelgrößen hergestellt werden, die in der Regel im Bereich von 1 µm bis etwa 500 µm liegen.

Die Verbindung der Formel (1) bildet eine weitere stabile kristalline Form IV, die bei höheren Temperaturen, bevorzugt bei einer Temperatur zwischen 70 °C und 155°C, besonders bevorzugt bei einer Temperatur zwischen 75 °C und 130 °C, noch weiter bevorzugt bei einer Temperatur zwischen 75 °C und 122 °C, thermodynamisch stabil ist und ebenfalls bei Normalbedingungen an Luft und unter Ausschluß von Luftfeuchtigkeit stabil ist.

Ein weiterer Gegenstand der Erfindung ist eine kristalline Form von 3-[2-Dimethylamino)methyl-(cyclohex-1-yl)-phenol Hydrochlorid der Formel (1), die ein charakteristisches Röntgen-Beugungsbild im Bereich von 2° bis 35° 2θ mit ausgeprägten charakteristischen Linien aufweist, ausgedrückt in 2Theta-Werte: 12.0 (m), 13.0 (m), 17.3 (m), 17.7 (m), 19.2 (s), 19.7 (m), 20.2 (m), 21.3 (m), 23.4 (m), 24.2 (m). 24.6 (m), 43.1 (vs), 44.2 (vs); nachfolgend als Form IV bezeichnet.

Ein weiterer Gegenstand der Erfindung ist eine kristalline Form IV von 3-[2-(Dimethylamino)methyl-(cyclohex-1-yl)]-phenol-HCl der Formel I, die ein charakteristisches Röntgen-Beugungsbild mit den nachstehenden ausgeprägten Reflexen aufweist.

| 2Theta | Intensität (relativ) |
|---|---|
| 10,73 | 6 |
| 12,04 | 42 |
| 12,38 | 32 |
| 13,02 | 59 |
| 13,78 | 6 |
| 14,71 | 6 |
| 14,96 | 5 |
| 15,62 | 21 |
| 15,91 | 8 |
| 16,65 | 12 |
| 17,28 | 42 |
| 17,73 | 47 |
| 18,78 | 23 |
| 19,22 | 86 |
| 19,67 | 39 |
| 20,16 | 51 |
| 20,59 | 17 |
| 21,30 | 47 |
| 22,07 | 78 |
| 23,40 | 43 |
| 24,25 | 47 |
| 24,56 | 41 |
| 25,04 | 14 |
| 25,44 | 19 |
| 25,72 | 31 |
| 27,22 | 22 |
| 27,82 | 35 |
| 28,53 | 14 |
| 29,13 | 24 |
| 29,62 | 31 |
| 29,99 | 21 |
| 30,58 | 12 |
| 31,33 | 21 |
| 31,57 | 18 |
| 31,91 | 16 |
| 32,45 | 18 |
| 32,85 | 13 |
| 33,33 | 10 |
| 33,75 | 15 |
| 34,48 | 25 |
| 34,92 | 18 |
| 35,46 | 16 |
| 36,44 | 12 |
| 37,36 | 18 |
| 38,15 | 18 |
| 38,58 | 20 |
| 39,67 | 14 |
| 40,56 | 17 |
| 41,05 | 15 |
| 41,63 | 14 |
| 41,85 | 14 |
| 42,57 | 15 |
| 43,14 | 96 |
| 43,25 | 60 |
| 44,24 | 100 |
| 45,99 | 23 |
| 46,81 | 16 |
| 49,06 | 22 |

Ein weiterer Gegenstand der Erfindung ist eine kristalline Form IV von 3-[2-Dimethylamino)methyl-(cyclohex-1-yl)-phenol Hydrochlorid der Formel (1), die ein charakteristisches Raman-Spektrum mit charakteristischen Banden aufweist, das durch die folgenden Wellenzahlen (cm⁻¹) beschrieben wird:

| Wellenzahl [cm⁻¹] | Intensität |
|---|---|
| 85 | S |
| 115 | S |
| 123 | S |
| 190 | VW |
| 243 | M |
| 337 | W |
| 386 | VW |
| 448 | VW |
| 476 | VW |
| 502 | VW |
| 534 | W |
| 574 | VW |
| 625 | W |
| 637 | VW |
| 709 | VW |
| 724 | VW |
| 748 | W |
| 761 | W |
| 794 | VW |
| 819 | VW |
| 833 | W |
| 844 | W |
| 858 | VW |
| 891 | VW |
| 913 | VW |
| 936 | VW |
| 957 | VW |
| 970 | VW |
| 988 | VW |
| 999 | VS |
| 1052 | W |
| 1071 | VW |
| 1083 | VW |
| 1104 | VW |
| 1123 | VW |
| 1160 | W |
| 1211 | VW |
| 1251 | VW |
| 1278 | W |
| 1293 | W |
| 1321 | W |
| 1337 | VW |
| 1366 | W |
| 1401 | VW |
| 1444 | W |
| 1463 | W |
| 1476 | VW |
| 1585 | W |
| 1614 | W |
| 2665 | VW |
| 2684 | VW |
| 2697 | VW |
| 2701 | VW |
| 2817 | W |
| 2853 | M |
| 2897 | M |
| 2914 | M |
| 2925 | M |
| 2938 | S |
| 2964 | M |
| 3017 | W |
| 3028 | W |
| 3043 | M |
| 3074 | VW |
| 3083 | VW |

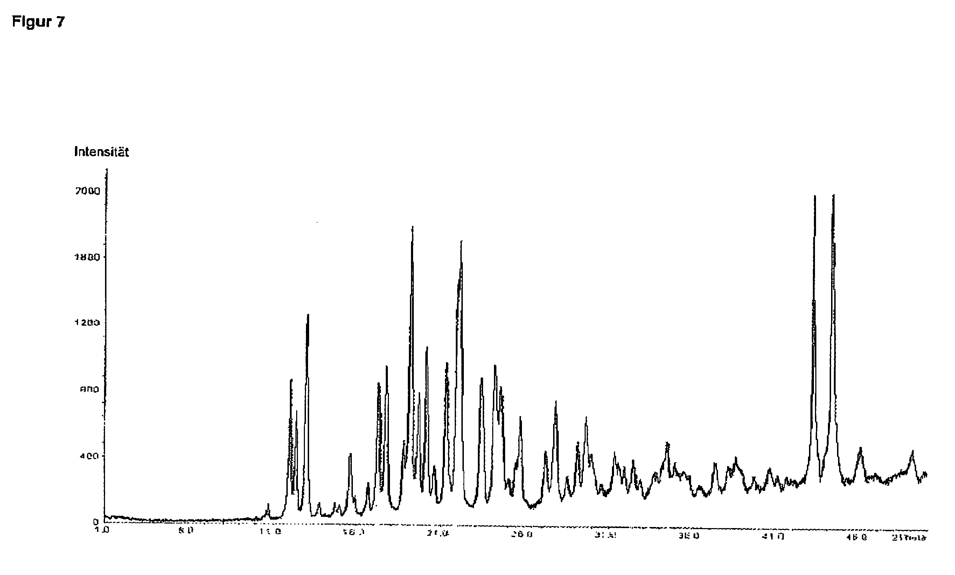

Ein weiterer Gegenstand der Erfindung ist eine kristalline Form IV von 3-[2-Dimethylamino)methyl-(cyclohex-1-yl)-phenol Hydrochlorid der Formel (1 ), die ein charakteristisches Röntgen-Beugungsbild wie in Figur 7 dargestellt besitzt.

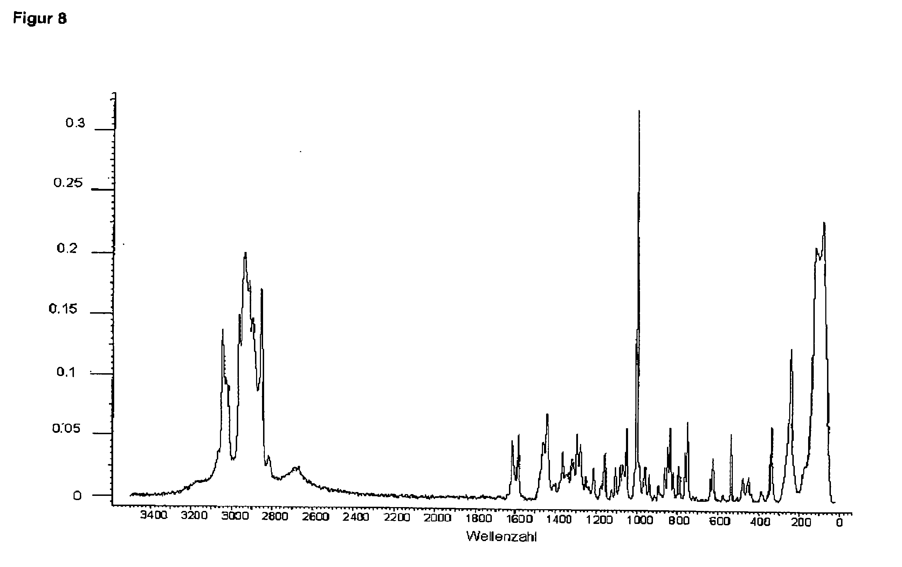

Ein weiterer Gegenstand der Erfindung ist eine kristalline Form IV von 3-[2-Dimethylamino)methyl-(cyclohex-1-yl)-phenol Hydrochlorid der Formel (1), die durch ein Raman-Spektrum wie in Figur 8 dargestellt charakterisiert, wird.

Die kristalline Form IV bildet in Anwesenheit von Luftfeuchtigkeit bei einer relativen Luftfeuchtigkeit > 50% irreversibel Hydrate der kristallinen Form V aus. Bei einer relativen Luftfeuchtigkeit > 95 % ergeben sich Hydrate mit einem Anteil von Kristallwasser bezogen auf das Gesamtgewicht des Hydrates im Bereich von 8 % bis 10%. Um die Bildung von Hydraten zu vermeiden, können die Verbindungen der kristallinen Form IV vorteilhafterweise in einer feuchtigkeitsarmen Umgebung beispielsweise in einem Gefäß über Phosphorpentoxid oder Molekularsieb gelagert werden. Ebenfalls ist eine Lagerung der kristallinen Form IV unter trockenem Schutzgas (zum Beispiel Stickstoff) empfehlenswert.

Der Schmelzpunkt liegt im Bereich zwischen 166 °C und 172 °C und die Schmelzenthalpie beträgt etwa 78 J/g, bestimmt mittels DSC bei einer Aufheizrate von 10°C/Minute. Polymorph IV kann als festes Pulver mit gewünschten mittleren Partikelgrößen hergestellt werden, die in der Regel im Bereich von 1 µm bis etwa 500 µm liegen.

Die Verbindung der Formel (1) bildet ferner stabile Hydrate der kristallinen Form V, die an Luft unter Normalbedingungen stabil sind.

Bevorzugt weisen die Hydrate der kristallinen Form V einen Anteil an Kristallwasser im Bereich von 1 % bis 10 %, besonders bevorzugt im Bereich von 5 % bis 9 %, ganz besonders bevorzugt im Bereich von 6 % bis 8.5 %, noch weiter bevorzugt im Bereich von 7 % bis 8 %, bezogen auf das Gewicht des Hydrates auf.

Ein weiterer Gegenstand der Erfindung ist eine kristalline Form von 3-[2-Dimethylamino)methyl-(cyclohex-1-yl)-phenol Hydrochlorid der Formel (1), die ein charakteristisches Röntgen-Beugungsbild im Bereich von 2° bis 35° 2θ mit ausgeprägten charakteristischen Linien aufweist, ausgedrückt in 2Theta-Werte: 11.4 (m), 12.1 (m), 16.7 (w), 19.2 (m), 19.4 (w), 20.1 (m), 21.1 (m), 22.4 (vs), 24.0 (m), 31.3 (w); nachfolgend als Form V bezeichnet.

Ein weiterer Gegenstand der Erfindung ist eine kristalline Form V von 3-[2-(Dimethylamino)methyl-(cyclohex-1-yl)]-phenol-HCl der Formel I, die ein charakteristisches Röntgen-Beugungsbild mit den nachstehenden ausgeprägten Reflexen aufweist.

| 2Theta | Intensität (relativ) |
|---|---|
| 9,06 | 6 |
| 11,47 | 60 |
| 12,15 | 36 |
| 13,43 | 9 |
| 14,32 | 11 |
| 14,89 | 6 |
| 16,72 | 17 |
| 17,42 | 4 |
| 18,12 | 5 |
| 18,82 | 6 |
| 19,23 | 46 |
| 19,44 | 31 |
| 20,07 | 50 |
| 20,68 | 8 |
| 21,09 | 50 |
| 21,88 | 8 |
| 22,42 | 100 |
| 23,00 | 5 |
| 23,53 | 6 |
| 24,04 | 46 |
| 24,37 | 33 |
| 24,54 | 26 |
| 24,58 | 27 |
| 25,05 | 7 |
| 25,89 | 29 |
| 26,52 | 7 |
| 26,99 | 6 |
| 27,32 | 12 |
| 27,91 | 13 |
| 28,65 | 10 |
| 30,11 | 13 |
| 30,93 | 17 |
| 31,29 | 20 |
| 32,43 | 17 |
| 32,96 | 11 |
| 33,74 | 5 |
| 34,11 | 6 |
| 34,46 | 7 |
| 34,84 | 9 |
| 35,89 | 7 |
| 36,31 | 6 |
| 36,88 | 7 |
| 37,38 | 12 |
| 37,71 | 8 |
| 38,06 | 5 |
| 38,99 | 7 |
| 40,01 | 7 |
| 40,72 | 5 |
| 41,70 | 4 |
| 42,35 | 5 |
| 42,94 | 8 |
| 43,86 | 5 |
| 44,15 | 5 |
| 44,70 | 4 |
| 45,07 | 6 |
| 45,65 | 6 |
| 46,24 | 5 |
| 47,31 | 6 |
| 48,23 | 4 |
| 49,12 | 4 |

Ein weiterer Gegenstand der Erfindung ist eine kristalline Form V von 3-[2-Dimethylamino)methyl-(cyclohex-1-yl)-phenol Hydrochlorid der Formel (1), die ein charakteristisches Raman-Spektrum mit charakteristischen Banden aufweist, das durch die folgenden Wellenzahlen (cm⁻¹) beschrieben wird:

| Wellenzahl [cm⁻¹] | Intensität |
|---|---|
| 96.38 | VS |
| 2.59 | W |
| 270.91 | W |
| 337.44 | W |
| 371.87 | VW |
| 448.81 | VW |
| 469.06 | VW |
| 507.14 | VW |
| 535.11 | W |
| 572.23 | VW |
| 624.30 | VW |
| 704.81 | VW |
| 51.58 | W |
| 94.48 | VW |
| 817.14 | VW |
| 837.39 | W |
| 857.16 | VW |
| 86.09 | VW |
| 15.01 | VW |
| 935.26 | VW |
| 952.62 | VW |
| 999.38 | S |
| 1053.38 | W |
| 1100.15 | VW |
| 1120.40 | VW |
| 1159.45 | VW |
| 1211.03 | VW |
| 1251.05 | VW |
| 1271.78 | VW |
| 1294.44 | W |
| 1308.42 | VW |
| 1336.87 | VW |
| 1355.19 | VW |
| 1368.69 | W |
| 1405.81 | VW |
| 1440.52 | W |
| 1459.81 | W |
| 1502.71 | VW |
| 1600.58 | W |
| 2667.03 | VW |
| 2702.70 | VW |
| 2728.74 | VW |
| 2811.66 | VW |
| 2855.05 | M |
| 2895.55 | M |
| 2934.60 | M |
| 2966.42 | M |
| 3032.47 | W |

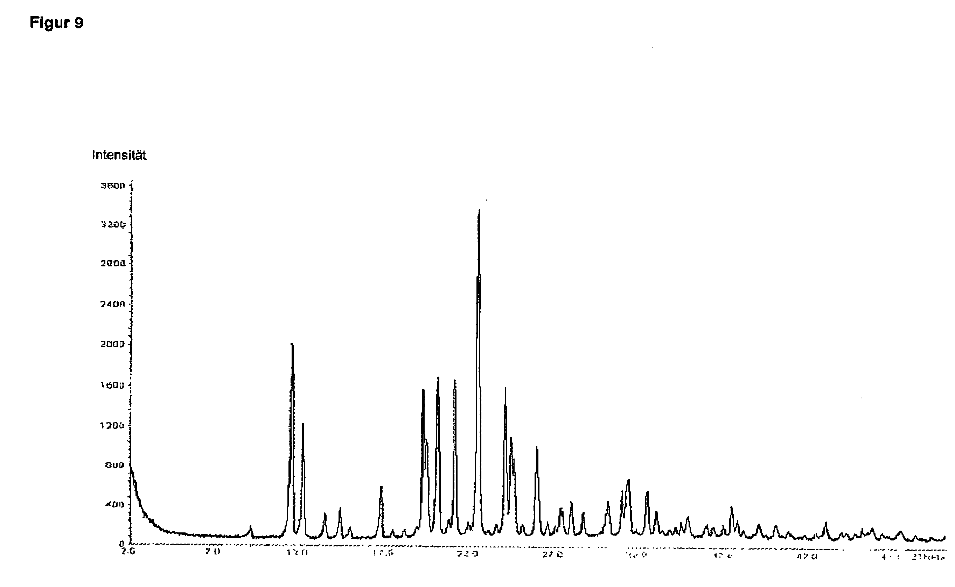

Ein weiterer Gegenstand der Erfindung ist eine kristalline Form V von 3-[2-Dimethylamino)methyl-(cyclohex-1-yl)-phenol Hydrochlorid der Formel (1), die ein charakteristisches Röntgen-Beugungsbild wie in Figur 9 dargestellt besitzt.

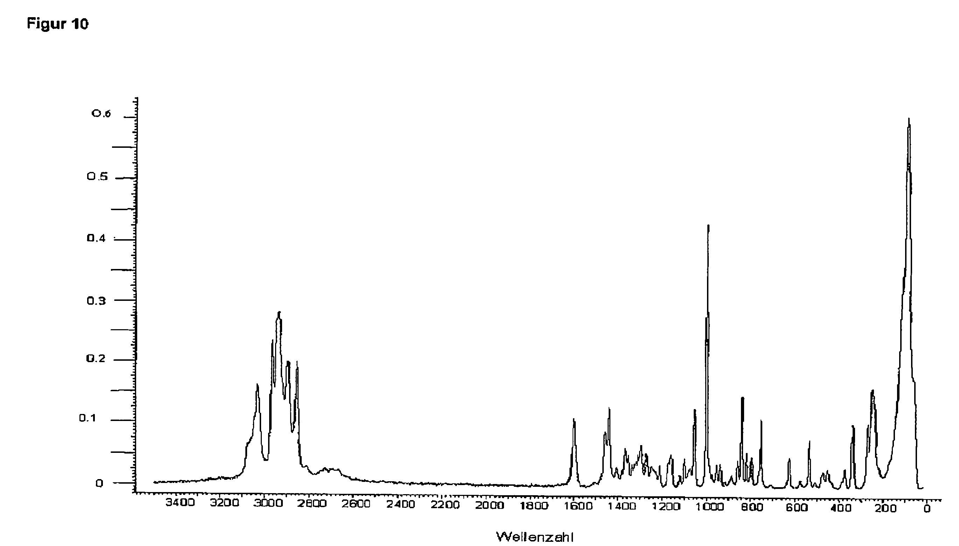

Ein weiterer Gegenstand der Erfindung ist eine kristalline Form V von 3-[2-Dimethylamino)methyl-(cyclohex-1-yl)phenol Hydrochlorid der Formel (1), die durch ein Raman-Spektrum wie in Figur 10 dargestellt charakterisiert, wird.

Die kristalline Form V Ist bei Raumtemperatur an Luft stabil und eignet sich daher besonders als Wirkstoff in pharmazeutischen Formulierungen.

Die kristalline Form V lässt sich nur schwer dehydratisieren. Der Einsatz von Vakuum und/oder hygroskopischen Reagenzien wie beispielsweise Phosphorpentoxid führt nur zu einer unvollständigen Abgabe von Wasser aus der kristallinen Form V. In Anwesenheit von Wasser, das beispielsweise In Form von Wasserdampf oder Luftfeuchtigkeit vorhanden sein kann, nimmt die zum Teil dehydratisierte Verbindung der kristallinen Form dieses schnell wieder auf. Die Verbindungen der kristallinen Form V verändern sich nicht unter erhöhtem Druck, beispielsweise bei einem Druck von 8000 bar für die Dauer von 60 Minuten, oder beim Mahlen, und man beobachtet keine Umwandlung In die kristallinen Formen I, II, III oder IV unter Einwirkung von höherem Druck.

Der Schmelzpunkt der Verbindungen der kristallinen Form V liegt im Bereich von 105 °C bis 115 °C und die Schmelzenthalpie beträgt etwa 77 J/g, bestimmt mittels DSC bei einer Aufheizrate von 10 °C/Minute.

Die Verbindung der Formel (1) bildet in solvatisierenden Lösungsmitteln wie beispielsweise Ethanol, Methanol, Methylethylketon, Isopropanol, n-Propanol, n-Octanol und Aceton stabile Solvate. Die Solvate sind zueinander und ebenfalls zum Hydrat mit der kristallinen Form V isomorph. Das Lösungsmittel lässt sich nicht oder nicht vollständig durch Vakuum entfernen.

Der Ersatz von Ethanol In einem entsprechenden Solvat von Verbindungen der Formel (1) gelingt durch Lagerung bei erhöhter Luftfeuchtigkeit, beispielsweise durch die Lagerung in Anwesenheit einer übersättigten wässrigen Lösung von Magnesiumnitrat oder einer übersättigten wässrigen Lösung Natriumchlorid für einen Zeitraum von wenigstens zwei Monaten, bevorzugt für einen Zeitraum von wenigstens vier Monaten.

Die polymorphen Formen I, II, III, IV und V können sich jeweils in andere kristalline Formen umwandeln. Beispielsweise können sich die polymorphen.Formen II, III und IV in die polymorphe Form I umwandeln; die polymorphen Formen III und IV können sich In die polymorphe Form II umwandeln; die polymorphe Formen III kann sich in die polymorphe Form IV umwandeln und die polymorphen Formen I und II können sich in die polymorphen Formen III oder IV umwandeln. Ein weiterer Gegenstand der Erfindung sind daher auch Mischungen der kristallinen Formen I, II, III, IV und V in an sich beliebigen Mischungsverhältnissen.

Die Kristallgitter der Formen I, II, III, IV und V sind deutlich voneinander verschieden, so dass die Raman-Spektren und Röntgenbaugungsbilder starke Unterschiede aufweisen. So weist die Form I einen Peak mit starker Intensität im Bereich von 19° 2θ auf, die Form III weist Peaks mit starker Intensität im Bereich von 7°, 14° und 21 ° 2θ auf und die Form V weist Peaks mit starker Intensität im Bereich von 12° und 22° 2θ auf.

Die polymorphen Formen I, II, III und IV können nach an sich bekannten Kristallisationsverfahren aus dem Salz 3-[2-(Dimethylamino)methyl-(cyclohex-1-yl)]-phenol Hydrochlorid hergestellt werden, zum Beispiel durch Rühren von Suspensionen (Einstellung von Phasengleichgewichten), Fällung, Umkristallisation, Verdampfung von Lösungsmitteln oder Kristallisation aus der Schmelze. Es können verdünnte, gesättigte oder übersättigte Lösungen verwendet werden, mit oder ohne Animpfen mit einem Kristallkeimbifdner. Die Temperaturen zur Bildung von Lösungen können bis zu 100 °C betragen. Die Kristallisation kann durch Kühlen auf etwa -100 °C bis 30 °C, und bevorzugt -30 °C bis 20 °C, initiert werden, wobei ein Abkühlen kontinuierlich oder stufenweise erfolgen kann. Zur Herstellung von Lösungen oder Suspensionen können amorphe oder kristalline Starimaterialien verwendet werden, um hohe Konzentrationen in Lösungen zu erzielen und andere kristalline Formen zu erhalten.

Ein weiterer Gegenstand der Erfindung ist daher ein Verfahren zur Herstellung der kristallinen Form I von 3-[2-(Dimethylamino)methyl-cyclohex-1-yl)]-phenol Hydrochlorid, das dadurch gekennzeichnet ist, dass
a) 3-[2-Dimethylamino)methyl-(cyclohex-1-yl)phenol Hydrochlorid in der kristallinen Form III und 3-[2-Dimethylamino)methy)-(cyclohex-1-yl)-phenol Hydrochlorid in der kristallinen Form IV oder 3-[2-Dimethylamino)methyl-(cyclohex-1-yl)-phenol Hydrochlorid in der kristallinen Form V in einem Lösungsmittel, bevorzugt in einem Mengenverhältnis zwischen 1:100 und 100:1, besonders bevorzugt in einem Mengenverhältnis zwischen 1:10 und 10:1, ganz besonders bevorzugt in einem Mengenverhältnis zwischen 1:5 und 5:1, bis zur vollständigen Bildung der kristallinen Form I gerührt werden, oder
b) 3-[2-Dimethylamino)methyl-(cyclohex-1-yl)-phenol Hydrochlorid in der kristallinen Form II und 3-[2-Dimethylamino)methyl-(cydohex-1-yl)-phonol Hydrochlorid in der kristallinen Form I in einem Lösungsmittel, bevorzugt in einem Mengenverhältnis zwischen 100:1 und 8:1, besonders bevorzugt in einem Mengenverhältnis zwischen 11:1 und 9:1, bis zur vollständigen Bildung der kristallinen Form I gerührt werden,
wobei die Temperatur in den Verfahren a) und b) höchstens 40 °C, bevorzugt höchstens 30 °C, besonders bevorzugt höchstens 25 °C, beträgt

Die Verfahren a) und b) können in Anwesenheit von Luft oder in Anwesenheit von inerten Gasen wie beispielsweise Stickstoff und Edelgasen durchgeführt werden. Luft als Umgebungsmedium ist aus wirtschaftlichen Gründen bevorzugt. Die relative Feuchtigkeit der Gase beträgt bevorzugt < 50 %, besonders bevorzugt < 20 %, ganz besonders bevorzugt < 5 %.

Die Dauer der Verfahren a) und b) hängt im Wesentlichen von der Größe der Kristalle und der Konzentration von 3-[2-Dimethylamino)methyl-(cyclohex-1-yl)-phenol Hydrochlorid ab und kann bevorzugt 1 bis 250 Stunden, besonders bevorzugt 3 bis 72 Stunden, ganz besonders bevorzugt 5 bis 25 Stunden, betragen. Die Konzentration von 3-[2-Dimethylamino)methyl-(cyclohex-1-yl)-phenol Hydrochlorid beträgt bevorzugt 0.5 % bis 50 %, besonders bevorzugt 2 % bis 30 %, ganz besonders bevorzugt 5 % bis 20 %, noch weiter bevorzugt 5 % bis 8 %, jeweils bezogen auf das Gewicht des Lösungsmittels

Nach der Isolierung kann der kristalline Rückstand in üblicher Weise getrocknet werden, wobei man zweckmäßig Temperaturen über 40 °C vermeidet.

Die Verfahren a) und b) werden bevorzugt in nicht-solvatisierenden Lösungsmitteln durchgeführt. Besonders bevorzugt sind Lösungsmittel ausgewählt aus der Gruppe umfassend aliphatische, cycloaliphatische und aromatische Kohlenwasserstoffe (Hexan, Heptan, Petrolether, Cyclohexan, Methylcyclohexan, Benzol, Toluol, Xylol), aliphatische Halogenkohlenwasserstoffe (Methylenchlorid, Chloroform, Di- und Tetrachlorethan), Nitrile (Acetonitril, Propionitril, Benzonitril), Ether (Diethylether, Dibutylether, tert-Butylmethylether, Ethylenglykoldimethylether, Ethylenglykoldiethylether, Diethylenglykoldimethylether, Tetrahydrofuran, Methyl-Tetrahydrofuran, Dioxan), langkettige Alkohole (Butanol, tert-Butanol, Pentanol, Oktanol, Dekanol) und Carbonsäureester und Lactone (Esslgsäurepropyl-, Essigsäureethyl- oder Essigsäuremethylester, Valerolacton). Ganz besonders bevorzugt ist das Lösungsmittel Ethylacetat.

Die Lösungsmittel können alleine oder in Mischung von wenigstens zwei Lösungsmitteln verwendet werden. Vorteilhaft verwendet man physiologisch unbedenkliche Lösungsmittel, die dem Fachmann geläufig sind. Nach der Isolierung kann das verwendete Lösungsmittel beziehungsweise Lösungsmittelgemisch in üblicher Weise mittels bekannter Trocknungsverfahren entfernt werden.

Ein weiterer Gegenstand der Erfindung ist eine kristalline Form I von 3-[2-Dimethylamino)methyl-(cyclohex-1-yl)phenol Hydrochlorid der Formel (1) erhältlich nach einem der vorstehend beschriebenen Verfahren.

Ein weiterer Gegenstand der Erfindung ist ebenfalls ein Verfahren zur Herstellung der kristallinen Form II von 3-[2-(Dimethylamino)methyl-(cyclohex-1-y)]-phenol Hydrochlorid, das dadurch gekennzeichnet ist, dass
a) 3-[2-Dimethylamino)methyl-(cyclohex-1-yl)-phenol Hydrochlorid in der kristallinen Form IV oder eine Mischung von 3-[2-Dimethylamino)methyl-(cyclohex-1-yl)-phenol Hydrochlorid in der kristallinen Form III und 3-[2-Dimethytamino)methyl-(cyclohex-1-yl)-phenol Hydrochlorid in der kristallinen Form V bei einer Temperatur zwischen 150 °C und 160 °C, bevorzugt bei einer Temperatur zwischen 154 °C und 158 °C, bis zur vollständigen Bildung der kristallinen Form II getempert wird, oder
b) 3-[2-Dimathylamino)methyl-(cyclohex-1-yl)-phenol Hydrochlorid in der kristallinen Form II und 3-[2-Dimethylamino)methyl-(cyclohex-1-yl)-pheno) Hydrochlorid in der kristallinen Form III oder 3-[2-Dimethylamino)methyl-(cyclohex-1-yl)-phenol Hydrochlorid in der kristallinen Form IV in einem Lösungsmittel, bevorzugt in einem Mengenverhältnis zwischen 1:100 und 1:8, besonders bevorzugt in einem Mengenverhältnis zwischen 1:11 und 1:9, bis zur vollständigen Bildung der kristallinen Form II gerührt wird, oder
c) eine Suspension der amorphen Form von 3-[2-Dimethylamino)methyl-(cyclohex-1-yl)-phenol Hydrochlorid in einem Lösungsmittel; bevorzugt in einem Lösungsmittel, das keine Solvate bildet; als Träger bei einer Temperatur zwischen 30 °C und 50 °C, bevorzugt bei einer Temperatur zwischen 35 °C und 45 °C, besonders bevorzugt bei einer Temperatur von 40 °C, bis zur vollständigen Bildung der kristallinen Form II gerührt wird; oder
d) 3-[2-Dimethylamino)methyl-(cydohex-1-yl)-phenol Hydrochlorid in der kristallinen Form III und 3-[2-Dimethylamino)methyl-(cyclohex-1-yl)-phenol Hydrochlorid in der kristallinen Form IV in einem Lösungsmittel, bevorzugt In einem Mengenverhältnis zwischen 1:100 und 100:1, besonders bevorzugt in einem Mengenverhältnis zwischen 1:10 und 10:1, ganz besonders bevorzugt in einem Mengenverhältnis zwischen 1:5 und 5:1, bis zur vollständigen Bildung der kristallinen Form II gerührt wird;
wobei die Temperatur in den Verfahren b) und d) höchstens 60 °C, bevorzugt höchstens 40 °C, besonders bevorzugt höchstens 30 °C, ganz besonders bevorzugt höchstens 25 °C beträgt

Die Verfahren b), c) und d) können in Anwesenheit von Luft oder in Anwesenheit von inerten Gasen wie beispielsweise Stickstoff und Edelgasen durchgeführt werden. Luft als Umgebungsmedium ist aus wirtschaftlichen Gründen bevorzugt. Die relative Feuchtigkeit der Gase beträgt bevorzugt < 50 %, besonders bevorzugt < 20 %, ganz besonders bevorzugt < 5 %.

Die Dauer der Verfahren b) und d) hängt im Wesentlichen von der Größe der Kristalle und der Konzentration von 3-[2-Dimethylamino)methyl-(cyclohex-1-yl)-phenol Hydrochlorid ab und kann bevorzugt 1 bis 250 Stunden, besonders bevorzugt 3 bis 72 Stunden, ganz besonders bevorzugt 5 bis 25 Stunden, betragen. Die Dauer des Verfahrens c) beträgt bevorzugt wenigstens 300 Stunden, besonders bevorzugt wenigstens 350 Stunden, ganz besonders bevorzugt wenigstens 400 Stunden. Die Konzentration von 3-[2-Dimethylamino)methyl-(cyclohex-1-yl)-phenol Hydrochlorid in den Verfahren b), c) und d) beträgt bevorzugt 0.5 % bis 50 %, besonders bevorzugt 2 % bis 30 %, ganz besonders bevorzugt 5 % bis 20 %, noch weiter bevorzugt 5 % bis 8 %, jeweils bezogen auf das Gewicht des Lösungsmittels.

Die Verfahren b), c) und d) werden bevorzugt in nicht-solvatisierenden Lösungsmitteln durchgeführt. Besonders bevorzugt sind Lösungsmittel ausgewählt aus der Gruppe umfassend aliphatische, cycloaliphatische und aromatische Kohlenwasserstoffe (Hexan, Heptan, Petrolether, Cyclohexan, Methylcyclohexan, Benzol, Toluol, Xylol), aliphatische Halogenkohlenwasserstoffe (Methylenchlorid, Chloroform, Di- und Tetrachlorethan), Nitrile (Acetonitril, Propionitril, Benzonitril), Ether (Diethylether, Dibutylether, tert-Butylmethylether, Ethylenglykoldimethylether, Ethylenglykoldiethylether, Diethylenglykoldimethylether, Tetrahydrofuran, Methyl-Tetrahydrofuran, Dioxan), langkettige Alkohole (Butanol, tert-Butanol, Pentanol, Oktanol, Dekanol) und Carbonsäureester und Lactone (Essigsäurepropyl-, Essigsäureethyl- oder Essigsäuremethylester, Valerolacton).

Ganz besonders bevorzugt ist das Lösungsmittel Ethylacetat.

Die Lösungsmittel können alleine oder in Mischung von wenigstens zwei Lösungsmitteln verwendet werden. Vorteilhaft verwendet man physiologisch unbedenkliche Lösungsmittel, die dem Fachmann geläufig sind. Nach der Isolierung kann das verwendete Lösungsmittel beziehungsweise Lösungsmittelgemisch in üblicher Weise mittels bekannter Trocknungsverfahren entfernt werden.

Ein weiterer Gegenstand der Erfindung ist eine kristalline Form II von 3-[2-Dimethylamino)methyl-(cyclohex-1-yl)-phenol Hydrochlorid der Formel (1) erhältlich nach einem der vorstehend beschriebenen Verfahren.

Ein weiterer Gegenstand der Erfindung ist ebenfalls ein Verfahren zur Herstellung der kristallinen Form III von 3-[2-(Dimethylamino)methyl-(cyclohex-1-yl)]-phenol Hydrochlorid, das dadurch gekennzeichnet ist, dass
a) 3-[2-Dimethylamino)methyl-(cyclohex-1-yl)-phenol Hydrochlorid in Form eines Ethanol- oder Aceton-Solvates in einem Lösungsmittel gelöst und gerührt und anschließend ausgefällt wird, oder
b) eine Suspension der amorphen Form von 3-[2-Dimethylamino)methyl-(cyclohex-1-yl)-phenol Hydrochlorid in einem Lösungsmittel; bevorzugt in einem Lösungsmittel, das keine Solvate bildet; als Träger bei einer Temperatur zwischen 30 °C und 80 °C, bevorzugt bei einer Temperatur zwischen 35 °C und 50 °C, besonders bevorzugt bei einer Temperatur von 40 °C, bis zur vollständigen Bildung der kristallinen Form III gerührt wird.

Im Verfahrensschritt a) können die kristallinen Formen I, II, IV, die amorphe Form von 3-[2-(Dimethylamino)methyl-(cyclohex-1-yl)]-phenol Hydrochlorid oder entsprechende Mischungen zur Herstellung von Lösungen verwendet werden. Die Konzentration an 3-[2-(Dimethylamino)methyl-(cyclohex-1-yl)]-phenol Hydrochlorid in der Lösung hängt von der gewählten Temperatur und vom Lösungsmittel ab. Die gelöste Menge kann bevorzugt von 0,5 % bis 50 %, besonders bevorzugt von 2 % bis 30 %, ganz besonders bevorzugt 3 % bis 25 % und noch weiter bevorzugt 5 % bis 20 % betragen, bezogen auf das Lösungsmittel. Die Temperatur zum Lösen kann bis zu 70 °C und bevorzugt bis zu 60 °C betragen. Bevorzugt wird Ethylacetat als Lösungsmittel zur Herstellung von Lösungen verwendet.

Die Fällung kann mittels Abkühlen, teilweiser oder vollständiger Entfernung des Lösungsmittels; Zugabe eines Lösungsmittels, worin 3-[2-(Dimethylamino)methyl-(cyclohex-1-yl)]-phenol Hydrochlorid nur eine geringe Löslichkeit aufweist wie beispielsweise Heptan, tert-Butylmethylether oder Ethylacetat und entsprechenden Mischungen; oder einer Kombination dieser Maßnahmen erfolgen. Abkühlen kann langsames Abkühlen oder auch Abschrecken auf Temperaturen bis -20 °C und bevorzugt bis 0°C bedeuten. Das Lösungsmittel kann durch Erwärmen, im Gasstrom, Anlegen von Vakuum oder einer Kombination dieser Maßnahmen entfernt werden. Erwärmen zum Entfernen von Lösungsmittel bedeutet in der Verfahrensstufe a) eine Temperatur von höchstens 40 °C und vorzugsweise höchstens 30°C.

Das Verfahren b) kann In Anwesenheit von Luft oder in Anwesenheit von inerten Gasen wie beispielsweise Stickstoff und Edelgasen durchgeführt werden. Luft als Umgebungsmedium ist aus wirtschaftlichen Gründen bevorzugt. Die relative Feuchtigkeit der Gase beträgt bevorzugt < 50 %, besonders bevorzugt < 40 %, ganz besonders bevorzugt < 20 %.

Die Dauer des Verfahrens b) hängt im Wesentlichen von der Größe der Kristalle und der Konzentration von 3-[2-Dimethylamino)methyl-(cyclohex-1-yl)-phonol Hydrochlorid ab und kann bevorzugt 1 bis 350 Stunden, besonders bevorzugt 10 bis 300 Stunden, ganz besonders bevorzugt 20 bis 300 Stunden, betragen. Die Konzentration von 3-[2-Dimethylamino)methyl-(cyclohex-1-yl)-phenol Hydrochlorid im Verfahren b) beträgt bevorzugt 0.5 % bis 50 %, besonders bevorzugt 2 % bis 30 %, ganz besonders bevorzugt 5 % bis 20 %, noch weiter bevorzugt 5 % bis 15 %, jeweils bezogen auf das Gewicht des Lösungsmittels. Nach der Isolierung kann der kristalline Rückstand in üblicher Weise getrocknet werden, wobei man zweckmäßig Temperaturen über 40 °C vermeidet.

Das Verfahren b) wird bevorzugt in nicht-solvatisierenden Lösungsmitteln durchgeführt. Besonders bevorzugt sind Lösungsmittel ausgewählt aus der Gruppe umfassend aliphatische, cycloaliphatische und aromatische Kohlenwasserstoffe (Hexan, Heptan, Petrolether, Cyclohexan, Methylcyclohexan, Benzol, Toluol, Xylol), aliphatische Halogenkohlenwasserstoffe (Methylenchlorid, Chloroform, Di- und Tetrachlorethan), Nitrile (Acetonitril, Propionitril, Benzonitril), Ether (Diethylether, Dibutylether, tert-Butylmethylether, Ethylenglykoldimethylether, Ethylenglykoldiethylether, Diethylenglykoldimethylether, Tetrahydrofuran, Methyl-Tetrahydrofuran, Dioxan), langkettige Alkohole (Butanol, tert-Butanol, Pentanol, Oktanol, Dekanol) und Carbonsäureester und Lactone (Essigsäurepropyl-, Essigsäureethyl- oder Essigsäuremethylester, Valerolacton).

Ganz besonders bevorzugt sind die Lösungsmittel Heptan, tert-Butylmethylether und Ethylacetat.

Ein weiterer Gegenstand der Erfindung ist eine kristalline Form III von 3-[2-Dimethylamino)methyl-(cyclohex-1-yl)-phenol Hydrochlorid der Formel (1) erhältlich nach einem der vorstehend beschriebenen Verfahren.

Ein weiterer Gegenstand der Erfindung ist ebenfalls ein Verfahren zur Herstellung der kristallinen Form IV von 3-[2-(Dimethylamino)methyl-(cyclohex-1-yl)]-phenol Hydrochlorid, das dadurch gekennzeichnet ist, dass
a) 3-[2-Dimethylamino)methyl-(cyclohex-1-yl)-phenol Hydrochlorid in der kristallinen Form III bei einer Temperatur zwischen 150 °C und 160 °C, bevorzugt bei einer Temperatur zwischen 154 °C und 158 °C, bis zur vollständigen Bildung der kristallinen Form IV getempert wird, oder
b) eine Suspension der amorphen Form von 3-[2-Dimethylamino)methyl-(cyclohex-1-yl)-phenol Hydrochlorid in einem Lösungsmittel; bevorzugt in einem Lösungsmittel, das keine Solvate bildet; als Träger bei einer Temperatur zwischen 40 °C und 120 °C, bevorzugt bei einer Temperatur zwischen 40 °C und 100 °C, besonders bevorzugt bei einer Temperatur zwischen 40 °C und 80 °C, bis zur vollständigen Bildung der kristallinen Form IV gerührt wird; oder
c) 3-[2-Dimethylamino)methyl-(cyclohex-1-yl)-phenol Hydrochlorid in der kristallinen Form IV und 3-[2-Dimethylamino)methyl-(cyclohex-1-yl)-phenol Hydrochlorid in der kristallinen Form III in einem Lösungsmittel, bevorzugt in einem Mengenverhältnis zwischen 1:100 und 1:8, besonders bevorzugt in einem Mengenverhältnis zwischen 1:11 und 1:9, bis zur vollständigen Bildung der kristallinen Form IV gerührt wird;
wobei die Temperatur in dem Verfahren c) höchstens 40 °C, bevorzugt höchstens 30 °C, besonders bevorzugt höchstens 25 °C, beträgt

Die Verfahren b) und c) können in Anwesenheit von Luft oder in Anwesenheit von inerten Gasen wie beispielsweise Stickstoff und Edelgasen durchgeführt werden. Luft als Umgebungsmedium ist aus wirtschaftlichen Gründen bevorzugt. Die relative Feuchtigkeit der Gase beträgt bevorzugt < 50 %, besonders bevorzugt < 40 %, ganz besonders bevorzugt < 20 %.

Die Dauer der Verfahren b) und c) hängt im Wesentlichen von der Größe der Kristalle und der Konzentration von 3-[2-Dimethylamino)methyl-(cyclohex-1-yl)-phenol Hydrochlorid ab und kann bevorzugt 1 bis 250 Stunden, besonders bevorzugt 10 bis 200 Stunden, ganz besonders bevorzugt 30 bis 150 Stunden, betragen. Die Konzentration von 3-[2-Dimethylamino)methyl-(cyclohex-1-yl)-phenol Hydrochlorid beträgt bevorzugt 0.5 % bis 50 %, besonders bevorzugt 0.5 % bis 20 %, ganz besonders bevorzugt 0.5 % bis 10 %, noch weiter bevorzugt 1 % bis 8 %, jeweils bezogen auf das Gewicht des Lösungsmittels. Nach der Isolierung kann der kristalline Rückstand in üblicher Weise getrocknet werden, wobei man zweckmäßig Temperaturen über 40 °C vermeidet.

Die Verfahren b) und c) werden bevorzugt in nicht-solvatisierenden Lösungsmitteln durchgeführt. Besonders bevorzugt sind Lösungsmittel ausgewählt aus der Gruppe umfassend aliphatische, cycloaliphatische und aromatische Kohlenwasserstoffe (Hexan, Heptan, Petrolether, Cyclohexan, Methylcyclohexan, Benzol, Toluol, Xylol), aliphatische Halogenkohlenwasserstoffe (Methylenchlorid, Chloroform, Di- und Tetrachforethan). Nitrile (Acetonitril, Propionitril, Benzonitril), Ether (Diethylether, Dibutylether, tert-Butylmethylether, Ethylenglykoldimethylether, Ethylenglykoldiethylether, Diethylenglykoldimethylether, Tetrahydrofuran, Methyl-Tetrahydrofuran, Dioxan), langkettige Alkohole (Butanol, tert-Butanol, Pentanol, Oktanol, Dekanol) und Carbonsäureester und Lactone (Essigsäurepropyl-, Essigsäureethyl- oder Essigsäuremethylester, Valerolacton). Ganz besonders bevorzugt ist das Lösungsmittel tert-Butylmethylether.

Die Lösungsmittel können alleine oder in Mischung von wenigstens zwei Lösungsmitteln verwendet werden. Vorteilhaft verwendet man physiologisch unbedenkliche Lösungsmittel, die dem Fachmann geläufig sind. Nach der Isolierung kann das verwendete Lösungsmittel beziehungsweise Lösungsmittelgemisch in üblicher Weise mittels bekannter Trocknungsverfahren entfernt werden.

Ein weiterer Gegenstand der Erfindung ist eine kristalline Form IV von 3-[2-Dimethylamino)methy-(cyclohex-1-yl)-phenol Hydrochlorid der Formel (1) erhältlich nach einem der vorstehend beschriebenen Verfahren.

Ein weiterer Gegenstand der Erfindung ist ebenfalls ein Verfahren zur Herstellung der kristallinen Form V von 3-[2-(Dimethylamino)methyl(cyclohex-1-yl)]-phenol Hydrochlorid, das dadurch gekennzeichnet ist, dass
a) 3-[2-Dimethylamino)methyl-(cyclohex-1-yl)-phenol Hydrochlorid in der kristallinen Form I, II, III oder IV an Luft stehen gelassen oder mit Wasserdampf behandelt wird, oder
b) eine Suspension der amorphen Form von 3-[2-Dimethylamino)methyl-(cyclohex-1-yl)-phenol Hydrochlorid in einer Mischung aus Wasser und ggf. wenigstens einem Lösungsmittel als Träger bei einer Temperatur zwischen 20 °C und 60 °C, bevorzugt bei einer Temperatur zwischen 20 °C und 30 °C, gerührt wird und anschließend das verbleibende Wasser bzw. Lösungsmittel entfernt wird.

Ein weiterer Gegenstand der Erfindung ist eine kristalline Form V von 3-[2-Dimethylamino)methyl-(cyclohex-1-yl)-phenol Hydrochlorid der Formel (1) erhältlich nach einem der vorstehend beschriebenen Verfahren.

3-[2-(Dimethylamino)methyl-(cyclohex-1-yl)]-phenol Hydrochlorid, Insbesondere 3-[2-(Dimethylamino)methyl-(cyclahex-1-yl)]-phenol Hydrochlorid in den kristallinen Form II und V, ist auf Grund seines günstigen Gesamteigenschaftsprofils hervorragend als Wirkstoff für pharmazeutische Zusammensetzungen und ganz besonders für schmerzstillende Medikamente geeignet. Demgemäß ist auch ein Erfindungsgegenstand die Verwendung von 3-[2-(Dimethylamino)methyl-(cyclohex-1-yl)]-phenol Hydrochlorid der Formel (1) als Wirkstoff in Arzneimitteln, bevorzugt als Wirkstoff in Schmerzmitteln. Bevorzugt sind auch hier, wie in der ganzen Anmeldung, Diastereomere oder Gemische von enantiomeren Diastereomeren mit trans-Konfiguration des Phenylringes und der Dimethylaminomethylgruppe (1 R,2R-beziehungsweise 1S,2S-Konfiguration), wobei das Enantiomer mit der absoluten Konfiguration (1R,2R) ganz besonders bevorzugt ist.

Ein weiterer Gegenstand der Erfindung ist eine pharmazeutische Zusammensetzung, enthaltend eine wirksame Menge 3-[2-(Dimethylamino)methyl-(cyclohex-1-yl)]-phenol Hydrochlorid der Formel (1) und einen pharmazeutischen Träger oder ein pharmazeutisches Verdünnungsmittel.

In der Zusammensetzung kann die Verbindung der Formel (1) als kristalline Form I, II, III, IV, V oder einer Mischung der Formen I, II, III, IV und V vorliegen. Vorzugsweise ist die kristalline Form II und/oder I und/oder V enthalten.

Besonders bevorzugt ist die kristalline Form II und/oder V enthalten.

Die Umwandlung der Verbindungen der kristallinen Form II in die kristalline Form I kann in pharmazeutischen Zusammensetzungen durch die Verwendung von pharmazeutisch annehmbare Zutaten und Komponenten oder durch die Verwendung von geeigneten, dem Fachmann bekannten Formulierungshilfsmitteln verhindert werden.

Die Menge an Verbindungen der Formel (1) hängt im wesentlichen vom Formulierungstyp und von der gewünschten Dosierung während des Zeitraums der Verabreichung ab. Die an den Patienten zu verabreichende Menge der jeweiligen Verbindungen der Formel (1) kann variieren und ist beispielsweise abhängig vom Gewicht oder Alter des Patienten sowie von der Applikationsart, der indikation und dem Schweregrad der Erkrankung. Bevorzugt werden 0,005 bis 5000 mg/kg, besonders bevorzugt 0,05 bis 500 mg/kg, ganz besonders bevorzugt 0.5 bis 140 mg/kg, noch weiter bevorzugt 2 bis 20 mg/kg Körpergewicht des Patienten wenigstens einer solchen Verbindung appliziert.

Bei oralen Formulierungen kann es sich um feste Formulierungen handeln, zum Beispiel Tabletten, Kapseln, Pillen und Pastillen. Bei oralen Formulierungen kann es sich auch um flüssige Formulierungen handeln, zum Beispiel Lösungen, Suspensionen, Sirupe oder Elexire. Flüssige und feste Formulierungen umfassen auch die Einarbeitung der Verbindungen der Formel I in feste oder flüssige Nahrungsmittel. Ferner umfassen Flüssigkeiten noch Lösungen für parenterale Applikationen wie zum Beispiel infusion oder Injektion.

Die Verbindungen der Formel I und die kristallinen Formen können direkt als Pulver (mikronisierte Teilchen), Granulate, Suspensionen oder Lösungen verwendet werden, oder sie können mit anderen pharmazeutisch annehmbaren Zutaten und Komponenten vermischt und dann pulverisiert werden, um die Pulver dann in Kapsein aus Hart- oder Weichgelatine zu füllen, Tabletten, Pillen oder Pastillen zu pressen, oder um die Pulver in einem Träger zu suspendieren oder zu lösen zur Herstellung von Suspensionen, Sirupen oder Elexieren. Tabletten, Pillen oder Pastillen können nach dem Pressen mit einem Überzug versehen werden.

Pharmazeutisch annehmbare Zutaten und Komponenten für die verschiedenen Formulierungstypen sind an sich bekannt. Es kann sich zum Beispiel um Bindemittel wie synthetische oder natürliche Polymere, Arzneiträger, Gleitmittel, Tenside, Süssmittel und Aromastoffe, Beschichtungsmittel, Konservierungsmittel, Farbstoffe, Verdickungsmittel, Hilfsmittel, antimikrobielle Mittel und Trägerfür die verschiedenen Formulierungstypen handeln.

Beispiele für Bindemittel sind Gummi Arabicum, Gummi Tragakant, Akaziengummi und biologisch abbaubare Polymere wie Homo- oder Copolyester von Dicarbonsäuren, Alkylendiolen, Polyalkylenglykolen und/oder aliphatischen Hydroxycarbonsäuren; Homo- oder Copolyamide von Dicarbonsäuren, Alkylendiaminen und/oder aliphatischen Aminocarbonsäuren; entsprechende Polyester-polyamid-copolymere, Polyanhydride, Polyorthoester, Polyphosphazene und Polycarbonate. Die biologisch abbaubaren Polymere können linear, verzeigt oder vernetzt sein. Spezifische Beispiele sind Polyglykolsäure, Polymilchsäure und Poly-d,l-milch-/ giykolsäure. Andere Beispiele für Polymere sind wasserlösliche Polymere wie zum Beispiel Polyoxaalkylene (Polyoxaethylen, Polyoxapropylen und Mischpolymere davon), Polyacrylamide und hydroxylalkylierte Polyacrylamide, Polymaleinsäure und Ester oder Amide davon, Polyacrylsäure und Ester oder Amide davon, Polyvinylalkohol und Ester oder Ether davon, Polyvinylimidazol, Polyvinylpyrrolidon und natürliche Polymere wie zum Beispiel Chitosan.

Beispiele für Arzneiträger sind Phosphate wie Dicalciumphosphat.

Beispiele für Gleitmittel sind natürliche oder synthetische Öle, Fette, Wachse oder Fettsäuresalze wie Magnesiumstearat.

Tenside (oberflächenaktive Mittel) können anionisch, kationisch, amphoter oder neutral sein. Beispiele für Tenside sind Lecithin, Phospholipide, Octylsulfat, Decylsulfat, Dodecylsulfat, Tetradecylsulfat, Hexadecylsulfat und Octadecylsulfat, Natriumoleat oder Natriumcaprat, 1-Acylaminoethan-2-sulfonsäuren wie 1-Octancylaminoethan-2-sulfonsäure, 1-Decanoylaminoethan-2-sulfonsäure, 1-Dodecanoylaminoethan-2-sulfonsäure, 1-Tetradecanoylaminoethan-2-sulfonsäure, 1-Hexadecanoylaminoethan-2-sulfonsäure und 1-Octadecanoylaminoethan-2-sulfonsäure, Gallensäuren, Salze und Derivate davon, wie zum Beispiel Cholsäure, Deoxycholsäure, Taurocholsäure, Taurodeoxycholsäure und Natriumglycocholate, Natriumcaprat, Natriumlaurat Natriumoleat, Natriumlaurylsulfat, Natriumcetylsulfat, sulfatiertes Castoröl, Natriumdioctylsulfosuccinat, Cocamidopropylbetain und Laurylbetain, Fettalkohole, Cholesterote, Glycerinmono- oder -distearat, Glycerinmono- oder-dioleat, Glycerinmono- oder -dipalmitat und Polyoxyethylenstearat.

Beispiele für Süssmittel sind Sucrose, Fructose, Lactose oder Aspartam.

Beispiele für Aromastoffe sind Pfefferminz, Öl von Wintergrün oder Fruchtaroma wie Kirschen- oder Orangenaroma.

Beispiele für Beschichtungsmittel sind Gelatine, Wachse, Schellack, Zucker oder biologisch abbaubare Polymere.

Beispiele für Konservierungsmittel sind Methyl- oder Propylparaben, Sorbinsäure, Chlorbutanol und Phenol.

Beispiele für Hilfsmittel sind Duftstoffe.

Beispiele für Verdickungsmittel sind synthetische Polymere, Fettsäuren, Fettsäuresalze, Fettsäureester und Fettalkohole.

Beispiele für flüssige Träger sind Wasser, Alkohole (Ethanol, Glycerol, Propylenglykol, flüssige Polyethylenglykole, Polytriazine und Öle. Beispiele für feste Träger sind Talk, Tonerden, mikrokristalline Cellulose, Siliziumdioxid, Aluminiumoxid und ähnliche Feststoffe.

Die erfindungsgemässe Zusammensetzung kann auch isotonische Mittel wie zum Beispiel Zucker, physiologische Puffer und Natriumchlorid enthalten.

Die erfindungsgemässe Zusammensetzung kann auch als Brausetablette oder Brausepulver formuliert sein, die sich in wässriger Umgebung zersetzt unter Zubereitung von Trinklösungen oder -suspensionen.

Ein Sirup oder Elexier kann die Verbindung der Formel I, einen Zucker wie Sucrose oder Fructose als Süssmittel, ein Konservierungsmittel (wie Methylparaben), einen Farbstoff und ein Geschmacksmittel (wie Aromastoffe) enthalten.

Bei der erfindungsgemässen Zusammensetzung kann es sich auch um Formulierungen mit verzögerter und kontrollierter Wirkstoffabgabe im Kontakt mit Körperflüssigkeiten des gastrointestinalen Traktes handeln, um einen im wesentlichen konstanten und effektiven Level des Wirkstoffs im Blutplasma zu erzielen. Die Verbindungen der Formel I können für diesen Zweck in eine Polymermatrix eines biologisch abbaubaren Polymers, eines wasserlöslichen Polymers oder beiden Polymeren eingebettet werden, gegebenenfalls zusammen mit einem geeigneten Tensid. Einbetten kann in diesem Zusammenhang die Einarbeitung von Mikropartikeln in die Polymermatrix bedeuten. Formulierungen mit verzögerter und kontrollierter Wirkstoffabgabe können auch mittels Enkapsulierung von dispergierten Mikropartikeln oder emulgierten Mikrotropfen über bekannte Beschithtungstechnclogien von Dispersionen und Emulsionen erhalten werden.

Die Verbindungen der Formel I können auch zusammen mit wenigstens einem weiteren pharmazeutischen Wirkstoff für Kombinationstherapien verwendet werden. Hierzu kann wenigstens ein weiterer Wirkstoff zusätzlich in der erfindungsgemässen Zusammensetzung dispergiert oder gelöst werden.

Gegenstand der Erfindung ist auch die Verwendung von 3-[2-(Dimethylamino)methyl-(cyclohex-1-yl)]-phenol-hydrochlorid der Formel I zur Herstellung einer pharmazeutischen Zusammensetzung, insbesondere für die Behandlung von Schmerzzuständen.

Ein weiterer Gegenstand der Erfindung ist ein Verfahren zur Behandlung von Schmerzzuständen, bei dem man einem unter Schmerzen leidenden Patienten eine wirksame Menge von 3-[2-(Dimethylamino)methyl-(cyclohex-1-yl)]-phenol-hydrochlorid der Formel verabreicht.

Bevorzugt eignet sich das erfindungsgemäße Arzneimittel (die erfindungsgemäße pharmazeutische Zusammensetzung) zur Prophylaxe und/oder Behandlung von Schmerz, vorzugsweise ausgewählt aus der Gruppe bestehend aus akutem Schmerz, chronischem Schmerz, neuropathischem Schmerz und visceralem Schmerz; von Migräne; Depressionen; neurodegenerativen Erkrankungen, vorzugsweise ausgewählt aus der Gruppe bestehend aus Morbus Parkinson, Morbus Alzheimer, Morbus Huntington und Multipler Sklerose; kognitiven Erkrankungen, vorzugsweise kognitiven Mangelzuständen, besonders bevorzugt des Aufmerksamkeit-Defizit-Syndroms (ADS), Panikattacken; Epilepsie; Husten; Harninkontinenz, Dlarrhöe; Pruritus; Schizophrenie; cerebralen Ischärnien; Muskelspasmen; Krämpfen; Störungen der Nahrungsaufnahme, vorzugsweise ausgewählt aus der Gruppe bestehend aus Bulimie, Kachexie, Anorexie und Fettleibigkeit; Alkohol- und/oder Drogen (insbesondere Nikotin- und/oder Kokain-) und/oder Medikamentenmißbrauch; Alkohol- und/oder Drogen (insbesondere Nikotin-und/oder Kokain-) und/oder Medikamentenabhängigkeit, vorzugsweise zur Prophylaxe und/oder Verminderung von Entzugserscheinungen bei Alkohol-und/oder Drogen (insbesondere Nikotin- und/oder Kokain-) und/oder Medikamentenabhängigkeit; Entwicklung von Toleranzerscheinungen gegenüber Medikamenten; insbesondere gegenüber Opioiden; des Magen-Ösaphagus-Reflux-Syndroms: zur Diurese; zur Antinatriurese; zur Beeinflussung des kardiovaskulären Systems; zur Anxiolyse; zur Vigilanzsteigerung; zur Libidosteigerung, zur Modulation der Bewegungsaktivität und zur Lokalanästhesie.

Besonders bevorzugt eignet sich das erfindungsgemäße Arzneimittel (die erfindungsgemäße pharmazeutische Zusammensetzung) zur Prophylaxe und/oder Behandlung von Schmerzen, vorzugsweise von akuten Schmerzen, chronischen Schmerzen, neuropathischen Schmerzen oder visceralen Schmerzen; Depressionen; Epilepsie; Morbus Parkinson; Alkohol- und/oder Drogen (insbesondere Nikotin-und/oder Kokain-) und/oder Medikamentenmißbrauch; Alkohol- und/oder Drogen (insbesondere Nikotin- und/oder Kokain-) und/oder Medikamentenabhängigkeit; vorzugsweise zur Prophylaxe und/oder Verminderung von Entzugserscheinungen bei Alkohol- und/oder Drogen (insbesondere Nikotin- und/oder Kokain-) und/oder Medikamentenabhängigkeit; der Entwicklung von Toleranzerscheinungen gegenüber Medikamenten, insbesondere gegenüber Opioiden, oder zur Anxiolyse.

Ganz besonders bevorzugt eignet sich das erfindungsgemäße Arzneimittel zur Prophylaxe und/oder Behandlung von Schmerzen, vorzugsweise von akuten Schmerzen, chronischen Schmerzen, neuropathischen Schmerzen oder visceralen Schmerzen.

Besonders bevorzugt ist die Verwendung wenigstens eines erfindungsgemäßen Salzes , jeweils ggf. in Form eines seiner reinen Stereoisomeren, insbesondere Enantiomeren oder Diastereomeren, seiner Racemate oder in Form einer Mischung von Stereoisomeren, insbesondere der Enantiomeren und/oder Diastereomeren, in einem beliebigen Mischungsverhältnis, sowie ggf. eines oder mehrerer pharmazeutisch verträglicher Hilfsstoffe zur Herstellung eines Arzneimittels zur Prophylaxe und/oder Behandlung von Schmerz, vorzugsweise ausgewählt aus der Gruppe bestehend aus akutem Schmerz, chronischem Schmerz, neuropathischem Schmerz und visceralem Schmerz, von Migräne, Depressionen, neurodegenerativen Erkrankungen, vorzugsweise ausgewählt aus der Gruppe bestehend aus Morbus Parkinson, Morbus Alzheimer, Morbus Huntington und Multipler Sklerose, kognitiven Erkrankungen, vorzugsweise kognitiven Mangelzuständen, besonders bevorzugt des Aufmerksamkelt-Defizit-Syndroms (ADS), Panikattacken, Epilepsie, Husten, Haminkontinenz, Diarrhöe, Pruritus, Schizophrenie, cerebralen Ischämien, Muskelspasmen, Krämpfen, Störungen der Nahrungsaufnahme, vorzugsweise ausgewählt aus der Gruppe bestehend aus Bulimie, Kachexie, Anorexie und Fettleibigkeit, Alkohol- und/oder Drogen (insbesondere Nikotin- und/oder Kokain-) und/oder Medikamentenmißbrauch; Alkohol- und/oder Drogen (insbesondere Nikotin-und/oder Kokain-) und/oder Medikamentenabhänglgkelt;vorzugsweise zur Prophylaxe und/oder Verminderung von Entzugserscheinungen bei Alkohol-und/oder Drogen (insbesondere Nikotin- und/oder Kokain-) und/oder Medikamentenabhängigkeit; Entwicklung von Toleranzerscheinungen gegenüber Drogen- und/oder Medikamenten, insbesondere gegenüber Opioiden, des Magen-Ösaphagus-Reflux-Syndroms, zur Diurese, zur Antinatriurese, zur Beeinflussung des kardiovaskulären Systems, zur Anxiolyse, zur Vigilanzsteigerung, zur Libidosteigerung, zur Modulation der Bewegungsaktivität und zur Lokalanästhesie.

Das erfindungsgemäße Arzneimittel kann als flüssige, halbfeste oder feste Arzneiform, beispielsweise in Form von Injektionslösungen, Tropfen, Säften, Sirupen, Sprays, Suspensionen, Tabletten, Patches, Kapseln, Pflastern, Zäpfchen, Salben, Cremes, Lotionen, Gelen, Emulsionen, Aerosolen oder In multipartikulärer Form, beispielsweise in Form von Pellets oder Granulaten, ggf. zu Tabletten verpreßt, in Kapseln abgefüllt oder in einer Flüssigkeit suspendiert, vorliegen und als solche auch verabreicht werden.

Neben wenigstens einem erfindungsgemäßen Salz, ggf. in Form seiner reinen Stereoisomeren, insbesondere Enantiomeren oder Diastereomeren, seines Racemates oder in Form von Mischungen der Stereoisomeren, insbesondere der Enantiomeren oder Diastereomeren, in einem beliebigen Mischungsverhältnis, enthält das erfindungsgemäße Arzneimittel (die erfindungsgemäße pharmazeutische Zusammenstzung) üblicherweise weitere physiologisch verträgliche pharmazeutische Hilfsstoffe, die bevorzugt ausgewählt werden können aus der Gruppe bestehend aus Trägermaterialien, Füllstoffen, Lösungsmitteln, Verdünnungsmitteln, oberflächenaktiven Stoffen, Farbstoffen, Konservierungsstoffen, Sprengmitteln, Gleitmitteln, Schmiermitteln, Aromen und Bindemitteln.

Die Auswahl der physiologisch verträglichen Hilfsstoffe sowie die einzusetzenden Mengen derselben hängt davon ab, ob das Arzneimittel (die pharmazeutische Zusammensetzung) oral, subkutan, parenteral, intravenös, intraperitoneal, intradermal, intramuskulär, intranasal, buccal, rectal oder örtlich, zum Beispiel auf Infektionen an der Haut, der Schleimhäute und an den Augen, appliziert werden soll. Für die orale Applikation eignen sich bevorzugt Zubereitungen in Form von Tabletten, Dragees, Kapseln, Granulaten, Pellets, Tropfen, Säften und Sirupen, für die parenterale, topische und inhalative Applikation Lösungen, Suspensionen, leicht rekonstituierbare Trockenzubereitungen sowie Sprays.

Geeignete perkutane Applikationszubereitungen sind auch Depotzubereitungen in gelöster Form oder in einem Pflaster, gegebenenfalls unter Zusatz von die Hautpenetration fördernden Mitteln.

Oral oder perkutan anwendbare Zubereitungsformen können die jeweiligen erfindungsgemäßen Salze verzögert freisetzen.

Die Herstellung der erfindungsgemäßen Arzneimittel erfolgt mittels üblichen, aus dem Stande der Technik wohl bekannten Mitteln, Vorrichtungen, Methoden und Verfahren, wie sie beispielsweise in "Remington's Pharmaceutical Sciences", Herausgeber A.R. Gennaro, 17. Auflage, Mack Publishing Company, Easton, Pa, 1985, insbesondere in Teil 8, Kapitel 76 bis 93 beschrieben sind Die entsprechende Beschreibung wird hiermit als Referenz eingeführt und gilt als Teil der Offenbarung.

Die an den Patienten zu verabreichende Menge der Jeweiligen erfindungsgemäßen Salze kann variieren und Ist beispielsweise abhängig vom Gewicht oder Alter des Patienten sowie von der Applikationsart, der indikation und dem Schweregrad der Erkrankung. Üblicherweise werden 0,005 bis 5000 mg/kg, vorzugsweise 0,05 bis 500 mg/kg Körpergewicht des Patienten wenigstens einer solchen Verbindung appliziert.

Die nachfolgenden Beispiele erläutern die Erfindung näher, ohne sie zu beschränken.

### Beispiele:

Bei allen DSC Messungen (wenn nicht anders angegeben) betragen die Aufheizraten 10°C / Minute; die angegebenen Temperaturen sind Peakmaxima.

### Beispiel 0.1:

### Synthese der Base 3-[2-Dimethylamino)methyl-(cyclohex-1-yl)-phenol

### Vorschrift a)

In einem 1000 mL Einhalskolben werden 52 g (183 mmol) der Vorstufe 1-(2-(3-methoxyphenyl)cyclohexyl)-N,N-dimethylmethanamin (Reinheit laut GC: 97,5% des trans Diastereomers) und 250 mL Bromwasserstoffsäure (47%, in Waser) zusammengegeben und 2 h unter Rückfluß mittels eines Magnetrührers gerührt. Nach beendeter Reaktion wurde der Bromwasserstoff unter Anlegen eines Vakuums (Wasserstrahlpumpe) abdestilliert. Aus dem Destillationsrückstand wurde mit Ethylacetat und wässriger Kaliumcarbonatlösung die Base 3-[2-Dlmethylamino)methyl-(cyclohex-1-yl)-phenol freigesetzt, dabei wurde die organische Phase mit MgSO₄ getrocknet und die Base 3-[2-Dimethylamino)methyl-(cyclohex-1-yl)-phenol als gelbliches Öl isoliert (Rohausbeute: 42 g, entspricht 98,6% der Theorie). Die Base 3-[2-Dimethylamino)methyl-(cyclohex-1-yl)-phenol wurde mit 250 mL Ethylacetat versetzt und über Nacht im Kühlschrank gelagert. Da kein Feststoff ausfiel wurde die Lösung erneut eingeengt, wobei ein starkes Aufschäumen beobachtet wurde.

### Vorschrift b)

In einem Einhalskolben wurden 125 g (440 mmol) der Vorstufe 1-(2-(3-methoxyphenyl)cyclohexyl)-N,N-dimethylmethanamin und 500 mL Bromwasserstoffsäure (47%, in Waser) zusammengegeben und 2 h unter Rückfluß mittels eines Magnetrührers gerührt. Nach beendeter Reaktion wurde der Bromwasserstoff unter Anlegen eines Vakuums (Wasserstrahlpumpe) abdestilliert. Der Destillationsrückstand wurde mit 250 mL Wasser versetzt und die Suspension mit 1000 mL Ethylacetat überschichtet. Mittels wässriger Natriumhydroxidlösung (c = 32% w/w) wurde unter Kühlung des Reaktionsgemisches mit Eis auf pH 8 eingestellt. Organische und wässerige Phase wurden separiert, die wässrige Phase dreimal mit jeweils 350 mL Ethylacetat extrahiert. Dabei wurde der pH-Wert kontrolliert und mittels wässriger Natriumhydroxidlösung (c = 32% w/w) auf einen Wert von mindestens pH 8 gehalten. Es wurde zwei weitere Male mit jeweils 150 mL Ethylacetat extrahiert und die organische Phase mittels MgSO₄ getrocknet. In einem Rotationsevaporator (Badtemperatur 44°C, Vakuum < 20 mbar) wurde das Lösungsmittel abgezogen. Die Ausbeute an Rohprodukt 3-[2-Dimethylamino)methyl-(cyclohex-1-yl)-phenol betrug 101 g. Das Rohprodukt 3-[2-Dimethylamino)methyl-(cyclohex-1-yl)-phenol wurde mit 100 mL Aceton versetzt und gerührt bis sich die hellbraune Substanz verfestigte. Im Anschluß wurde das Material abgesaugt und mit wenig Diethylether gewaschen. Die Ausbeute betrug 48% bezogen auf die eingesetze Menge an Rohprodukt.

### Beispiel 0.2 Herstellung von 3-[2-Dimethylamino)methyl-(cyclohex-1-yl)-phenol hydrochlorid

Zur Darstellung des Hydrochloridsalzes wurden 42 g der Base 3-[2-Dimethylamino)methyl-(cyclohex-1-yl)-phenol mit 210 ml Methyl-Ethylketon verstetzt Anschliessend wurden 2 ml Wasser und 23 ml Trimethylchlorsilan, gelöst in 45 mL Methyl-Ethyl-Keton, zugegeben. Das Reaktionsgemisch wurde durch Kühlung mit Eis auf 0°C abgekühlt und über Nacht kalt (im Kühlschrank bei ca. 4°C) aufbewahrt. Der ausgefallene Feststoff wurde mittels einer Nutsche unter Anlegen eines Vakuums abgesaugt und mit mit 30 mL Aceton nachgewaschen. Die Ausbeute an 3-[2-Dimethylamino)methyl-(cyclohex-1-yl)-phenol Hydrochlorid betrug 49 g (entspricht 100 % der Theorie). Laut NMR- und GC-Analytik > 95 % trans-Diastereomer Der Schmelzpunk betrug 122 -126 °C

### Beispiel 1a:

### Herstellung von (1R,2R)-3-[2-Dimethylamino)methyl-(cyclohex-1-yl)-phenol Hydrochlorid

Zur Darstellung des Hydrochloridsalzes wurden 48,5 g (208 mmol) der Base 3-[2-Dimethylamino)mothyl-(cyclohex-1-yl)-phenol dargestellt wie in Beispiel 0.1 nach Vorschrift b) mit 250 ml Aceton versetzt. Anschliessend wurden 2 ml Wasser und 27 ml Trimethylchlorsilan (TMCS), gelöst in 125 mL Aceton, zugegeben. Der ausgefallene Feststoff wurde mittels einer Nutsche unter Anlegen eines Vakuums abgesaugt und mit Diethylether nachgewaschen.

Die Ausbeute betrug 53 g. (nach GC Analyse 100% des trans-Diastereomers).

Weitere Chargen des Hydrochloridsalzes 3-[2-Dimethylemino)methyl-(cyclohex-1-yl)-phenol Hydrochlorid wurden anlog wie beschrieben hergestellt.

| Charge | Vorstufe /g | HBr (47%) /mL | Base /g | Aceton /mL | Wasser /mL | TMCS (in Aceton) /mL | Ausbeute /g | trans Diastereomer /% |
|---|---|---|---|---|---|---|---|---|
| 1-12 | 129 | 520 | 109 | 550 | 4,5 | 55,7 (275) | 124 | 98,8 |
| 1-13 | 143 | 600 | 117 | 585 | 5 | 63,5 (290) | 119 | 98,6 |
| 1-14 | 121 | 520 | 101 | 500 | 4 | 55 (250) | 119 | 98,7 |
| 1-15 | 163 | 650 | 130 | 650 | 6 | 72 (325) | 153 | 97,4 |
| 1-16 | 148 | 600 | 121 | 610 | 5,5 | 65,5 (300) | 138 | 98,8 |

Die Feststoffe der Chargen 1-12 bis 1-16 wurden vereinigt und innig vermengt.

Zur Entfernung von Restlösemittel (Aceton und Diethylether) wurde vorgetrocknet (1 d, Raumtemperatur, Vakuum <150 mmbar) und anschliessend 6 Tage bei 70°C und einem Vakuum von < 20 mbar über Sicacide getrocknet.

Nach GC Analytik betrug der Anteil an Restlösemittel (Aceton, Diethylether) jeweils weniger als 200 ppm.

### Beispiel 1b:

### Herstellung von (1 R,2R)-3-[2-Dimethylamino)methyl-(cyclohex-1-yl)-phenol Hydrochlorid

In einer 100L Doppelmantelreaktionsanlage mit elektrischem Impellerrüher, Pt100 Temperaturmeßeinrichtung und ölbasierendem Kühl-/Heizsystem werden 37 kg (137.12 mol) (1R,2R)-3-(2-Dimethylaminomethyl-cyclohexyl)-phenol Hydrochlorid bei einer Rührdrehzahl von 100 U/min in 55 L Wasser gelöst. Die Lösung wird auf 40 °C - 60 °C erwärmt, bis eine klare Lösung entsteht. Bei reduziertem Druck (ca. 30 - 50 mbar) werden 38L - 41 L des Wassers entfernt.

Die Lösung wird bei 7°C ca. 16h gerührt. Die erhaltene Suspension wird über eine Zentrifuge abgetrennt. Das Produkt wird im Trockenschrank bei 45°C 18h im Vakuum bis zu einem Endruck von 130mbar getrocknet. Man erhält 25,9 kg (70% der Theorie) (1 R,2R)-3-(2-Dimethylaminomethyl-cyclohexyl)-phenol Hydrochlorid mit einem Restwassergehalt von 5,9%.

### Beispiel 2:

### Darstellung von amorphem (1R,2R)-3-[2-Dimethylamino)methyl-(cyclohex-1-yl)-phenol Hydrochlorid

### Beispiel 2.1: Gefriertrocknung

500.4 mg des gemäß Beispiel 1 b hergestellten Hydrochlorids werden in 5 ml Wasser gelöst und danach auf - 74 °C abgeschreckt. Dann wird bei dieser Temperatur und einem Druck von < 0.1 mbar während 20 Stunden gefriergetrocknet. Man erhält quantitativ einen festen, weißen Rückstand, der nach Auswertung des Raman-Spektrums amorph ist.

### Beispiel 2.2: Gefriertrocknung

212.5 mg des gemäß Beispiel 1 b hergestellten Hydrochlorids werden in 2 ml Wasser gelöst und danach auf -89 °C abgeschreckt. Dann wird bei dieser Temperatur und einem Druck von < 0.01 mbar während 66 Stunden gefriergetrocknet. Man erhält quantitativ einen festen, weißen Rückstand, der nach Auswertung des Raman-Spektrums amorph ist.

### Beispiel 2.3: Gefriertrocknung

651.5 mg des gemäß Beispiel 1b hergestellten Hydrochlorids werden in 6.5 ml Wasser gelöst und danach auf -80 °C abgeschreckt. Dann wird bei dieser Temperatur und einem Druck von 0.6 mbar während 21 Stunden gefriergetrocknet. Man erhält quantitativ einen festen, weißen Rückstand, der nach Auswertung des Raman-Spektrums amorph ist.

### Beispiel 3:

### Darstellung von (1R,2R)-3-[2-Dimethylamino)methyl-(cyclohex-1-yl)-phenol Hydrochlorid als kristalline Form I

### Beispiel 3.1:

209.7 mg des gemäß Beispiel 1a hergestellten Hydrochlorids werden in 6 mL Ethylacetat suspendiert und für zehn Tage bei einer Umdrehungszahl des Rührers von etwa 600 Upm (Umdrehungen pro Minute) und einer Temperatur von 23°C bis 28°C gerührt, wobei die Temperatur die ersten zwei Tage 23°C, die nächsten zwei Tage 28°C und für die restliche Dauer 23°C beträgt. Der weiße Feststoff wird abgetrennt und analysiert. Gemäß Raman-Spektrum werden nur Banden der kristallinen Form I gemessen.

### Beispiel 3.2:

101.4 mg des gemäß Beispiel 1a hergestellten Hydrochlorids werden in 3 mL Ethylacetat suspendiert, mit 14.5 mg des Hydrochlorids in der kristallinen Form I versetzt und für zehn Tage bei einer Umdrehungszahl des Rührers von etwa 600 Upm (Umdrehungen pro Minute) und einer Temperatur von 23°C gerührt. Der weiße Feststoff wird durch Vakuumfiltration (5 Minuten) abgetrennt und an der Luft getrocknet. Gemäß Röntgen-Pulverdiffraktogramm werden nur Signale der kristallinen Form I gemessen. Mittels Differential Scanning Calorimetrie (DSC, Erwärmungsrate 10°C/Minute) wird ein Schmelzpunkt von etwa 150°C ermittelt. Ein endothermer Peak wird im Bereich von 121°C bis 122°C beobachtet.

### Beispiel 4:

### Darstellung von (1R,2R)-3-[2-Dimethylamino)methyl-(cyclohex-1-yl)-phenol Hydrochlorid als kristalline Form II

### Beispiel 4.1:

191.8 mg des gemäß Beispiel 1 b hergestellten Hydrochlorids werden für 23.5 Stunden in einem offenen Gefäß bei 155°C getempert. Gemäß Röntgen-Pulverdiffraktogramm werden nur Signale der kristallinen Form II gemessen. Mittels Differential Scanning Calorimetrie (DSC, Erwärmungsrate 10 °C/Minute) wird ein Schmelzpunkt von etwa 177°C ermittelt.

### Beispiel 4.2:

600 mg des Hydrochlorids der kristallinen Form III werden mit 100 mg des Hydrochlorids in der kristallinen Form II versetzt, innig vermengt und in 10 mL Ethylacetat suspendiert. Die Suspension wird für sechs Tage bei Raumtemperatur gerührt. Der weiße Feststoff wird durch Vakuumfiltration abgetrennt und 1,5 h in einem Trockenschrank bei einer Temperatur von 45°C und einem Vakuum <150 mbar getrocknet.

Der so erhaltene Feststoff weist gemäß DSC Analytik (Heizrate 10 K/Minute) eine Endothermie bei 177.2°C auf, welche der kristallinen Form II zuzuordnen ist.

### Beispiel 4.3:

4.0 g Hydrochlorid der kristallinen Form V werden in einem Gefäß ohne Lösungsmittel bei einer Temperatur von 154°C (Temperatur des Ölbads 154°C bis 162 °C) gerührt.

Der erhaltene Feststoff weist gemäß Raman-Spektrum nur Banden der kristallinen Form II auf.

### Beispiel 5:

### Darstellung von (1R,2R)-3-[2-Dimethylamino)methyl-(cyclohex-1-yl)-phenol Hydrochlorid als kristalline Form III

### Beispiel 5.1:

76.9 mg des gemäß Beispiel 2 hergestellten amorphen Hydrochlorids werden in 1 mL Ethylacetat suspendiert und für drei Tage bei einer Umdrehungszahl des Rührers von etwa 400 Upm (Umdrehungen pro Minute) und einer Temperatur von 25°C, für einen Tag bei 40°C, für einen Tag bei 60°C, für einen Tag bei 50°C, für einen Tag bei 45°C, für einen Tag bei 70°C und für 9 Tage bei 75°C gerührt, wobei die Umdrehungszahl ab dem dritten Tag jeweils 600 Upm beträgt. Der weiße Feststoff wird durch Vakuumfiltration (ca. 5 Minuten) abgetrennt und an der Luft getrocknet. Gemäß Röntgen-Pulverdiffraktogramm werden nur Signale der kristallinen Form III gemessen.

### Beispiel 5.2:

70.5 mg des gemäß Beispiel 2 hergestellten amorphen Hydrochlorids werden in 1.5 mL tert-Butylmethylether suspendiert und für sieben Tage bei einer Umdrehungszahl des Rührers von etwa 600 Upm (Umdrehungen pro Minute) und einer Temperatur von 40°C gerührt. Der weiße Feststoff wird durch Vakuumfiltration (ca. 5 Minuten) abgetrennt und an der Luft getrocknet. Gemäß Raman-Spektrum werden nur Banden der kristallinen Form III gemessen. Mittels Differential Scanning Calorimetrie (DSC, Erwärmungsrate 10°C/Minute) wird eine Glasumwandlungstemperatur zwischen 66 °C und 67°C und ein Schmelzpunkt von etwa 155°C ermittelt. Ein weiterer endothermer Peak wird bei 88 °C beobachtet.

### Beispiel 5.3:

147.8 mg des gemäß Beispiel 2 hergestellten amorphen Hydrochlorids werden in 1.5 mL Ethylacetat suspendiert und für einen Tage bei einer Umdrehungszahl des Rührers von etwa 600 Upm (Umdrehungen pro Minute) und einer Temperatur von 40 °C gerührt. Der weiße Feststoff wird durch Vakuumfiltration (ca. 5 Minuten) abgetrennt und an der Luft getrocknet. Gemäß Raman-Spektrum werden nur Banden der kristallinen Form III gemessen.

### Beispiel 5.4:

1 g eines Aceton- oder Ethanolsolvates des Hydrochlorids werden in 18 mL Ethylacetat suspendiert und bei einer Temperatur von 23°C gerührt. Der weiße Feststoff wird durch Vakuumfiltration (5 Minuten) abgetrennt und an der Luft getrocknet. Gemäß Raman-Spektrum werden nur Banden der kristallinen Form III gemessen.

### Beispiel 6:

### Darstellung von (1R,2R)-3-[2-Dimethylamino)methyl-(cyclohex-1-yl)-phenol Hydrochlorid als kristalline Form IV

### Beispiel 6.1:

178.2 mg des gemäß Beispiel 2 hergestellten amorphen Hydrochlorids werden in 3.6 mL tert-Butylmethylether suspendiert und für einen Tag bei einer Umdrehungszahl des Rührers von etwa 600 Upm (Umdrehungen pro Minute) und einer Temperatur von 40°C gerührt. Der weiße Feststoff wird durch Vakuumfiltration (ca. 5 Minuten) abgetrennt und an der Luft getrocknet. Gemäß Röntgen-Pulverdiffraktogramm werden nur Linien der kristallinen Form IV gemessen. Mittels Differential Scanning Calorimetrie (DSC, Erwärmungsrate 10°C/Minute) wird eine geringe Exothermie bei ca. 122°C beobachtet, die auf eine monotrope Umwandlung in eine andere Form hindeutet. Im weiteren Verlauf des DSC Experimentes wird zunächst ein endothermer Peak bei etwa 162°C und ein weiterer bei etwa 171°C beobachtet.

### Beispiel 6.2:

153.2 mg des gemäß Beispiel 2 hergestellten amorphen Hydrochlorids werden in 3.5 mL Heptan suspendiert und für 26 Tage bei einer Umdrehungszahl des Rührers von etwa 600 Upm (Umdrehungen pro Minute) und einer im Bereich von 40 °C bis 90 °C gerührt, wobei die Temperatur die ersten acht Tage 40 °C, die nächsten sechs Tage 50 °C, einen Tag 75°C, drei Tage 80°C und acht Tage 90°C beträgt. Der weiße Feststoff wird durch Zentrifugieren (10 Minuten bei 10000 Umdrehungen) abgetrennt und an der Luft getrocknet. Gemäß Raman-Spektrum werden nur Banden der kristallinen Form IV gemessen.

### Beispiel 6.3:

4.0 g Hydrochlorid der kristallinen Form III werden in einem 100 mL Rundkolben ohne Lösungsmittel für fünf Stunden bei einer Temperatur von 154°C (Temperatur des Ölbads 154°C bis 162 °C) gerührt.

Der erhaltene Feststoff weist gemäß DSC Analytik (Heizrate 10 K/Minute) eine Endothermie bei 168.8 °C auf, welche der kristallinen Form IV zuzuordnen ist.

### Beispiel 7:

### Darstellung von (1R,2R)-3-[2-Dimethylamino)methyl-(cyclohex-1-yl)-phenol Hydrochlorid als kristalline Form V

### Beispiel 7.1:

53.7 mg des gemäß Beispiel 1b hergestellten Hydrochlorids werden in 1 mL Ethanol und Wasser (Volumenverhältnis 1:1) bei Raumtemperatur gelöst und bei Raumtemperatur an der Luft stehen gelassen bis das Lösungsmittelgemisch vollständig verdampft ist und ein Feststoff verbleibt. Gemäß Röntgen-Pulverdiffraktogramm werden nur Linien der kristallinen Form V gemessen. Bei dem festen Rückstand handelt es sich daher um die kristalline Form V.

### Beispiel 8: Stabilität bei Raumtemperatur

### Beispiel 8.1:

51.4 mg des Hydrochlorids in der kristallinen Form I und 49.8 mg des Hydrochlorids In der kristallinen Form II werden in 2 mL Ethylacetat suspendiert und für elf Tage bei einer Temperatur von 25°C gerührt. Der Feststoff wird durch Vakuumfiltration abgetrennt und an der Luft getrocknet. Gemäß Raman-Spektrum enthält die Probe überwiegend die kristalline Form I und geringe Anteile der Form V.

### Beispiel 8.2:

33.6 mg des Hydrochlorids in der kristallinen Form III und 31.5 mg des Hydrochlorids in der kristallinen Form IV werden in 2 mL Ethylacetat suspendiert und für zwölf Tage bei einer Temperatur von 23°C gerührt. Der Feststoff wird durch Vakuumfiltration abgetrennt und an der Luft getrocknet. Gemäß Raman-Spektrum werden nur Banden der kristallinen Form I gemessen.

### Beispiel 8.3:

30.6 mg des Hydrochlorids in der kristallinen Form III und 30.7 mg des Hydrochlorids in der kristallinen Form II werden in 2 mL Ethylacetat suspendiert und für drei Tage bei einer Temperatur von 23°C gerührt. Der weiße Feststoff wird durch Vakuumfiltration abgetrennt und an der Luft getrocknet. Gemäß Raman-Spektrum werden nur Banden der kristallinen Form II gemessen.

### Beispiel 8.4:

31.1 mg des Hydrochlorids In der kristallinen Form IV und 28.9 mg des Hydrochlorids in der kristallinen Form II werden in 2 mL Ethylacetat suspendiert und für 32 Tage bei einer Temperatur von 23°C gerührt. Der weiße Feststoff wird durch Vakuumfiltration abgetrennt und an der Luft getrocknet. Gemäß Raman-Spektrum werden zunächst nur Banden der kristallinen Form **II** gemessen. Nach Ablauf der ersten drei Versuchstage werden die ersten Spuren der kristallinen Form I und nach 32 Tagen wird fast ausschließlich die kristalline Form I detektiert.

Aus den Beispielen 8.1 bis 8.4 ergibt sich für die Stabilität der kristallinen Verbindungen I, II, III und IV bei Raumtemperatur unter Bedingungen zur Vermeidung der Bildung von Solvaten diese Reihenfolge: I > II > III=IV.

### Beispiel 9: Wasseraufnahme

Die Wasseraufnahme wird mittels dynamischer Wasserdampfaufnahme (Dynamic Vapor Sorption, DVS) mit dem Gerät DVS-1 der Firma Surface Measurement Systems Ltd. ermittelt. Die Probe wird in einem Platintiegel an der Spitze einer Mikrowaage plaziert. Dann wird die Probe zunächst bei 50% relativer Luftfeuchtigkeit equilibriert und dann einem vordefinierten Meßprogramm unterzogen. Die Temperatur beträgt 25 °C. Bestimmt wird die Gewichtsänderung der Probe.

### A) Kristalline Form I

Die kristalline Form nimmt bei einer relativen Luftfeuchtigkeit > 50 % sehr schnell Wasser auf. Wenn die relative Luftfeuchtigkeit auf 0 % verringert wird, reduziert sich der Wassergehalt der Probe auf 3.2 Gew.-%. Der Wassergehalt bei 50 % relativer Luftfeuchtigkeit am Ende des Meßzykluses liegt bei 7.2 % und das aufgenommene Raman-Spektrum entspricht dem Raman-Spektrum der kristallinen Form V.

### B) Kristalline Form II

Die kristalline Form nimmt bei einer relativen Luftfeuchtigkeit > 75 % sehr schnell Wasser auf. Wenn die relative Luftfeuchtigkeit auf 0 % verringert wird, reduziert sich der Wassergehalt der Probe auf 3.3 Gew.-%. Der Wassergehalt bei 50 % relativer Luftfeuchtigkeit am Ende des Meßzykluses liegt bei 7.9 % und das aufgenommene Raman-Spektrum entspricht dem Raman-Spektrum der kristallinen Form V.

### C) Kristalline Form III

Die kristalline Form nimmt bei einer relativen Luftfeuchtigkeit > 55 % sehr schnell Wasser auf. Wenn die relative Luftfeuchtigkeit auf 0 % verringert wird, reduziert sich der Wassergehalt der Probe auf 3.1 Gew.-%. Der Wassergehalt bei 50 % relativer Luftfeuchtigkeit am Ende des Meßzykluses liegt bei 7.8 % und das aufgenommene Raman-Spektrum entspricht dem Raman-Spektrum der kristallinen Form V.

### D) Kristalline Form IV

Die kristalline Form nimmt bei einer relativen Luftfeuchtigkeit > 60 % sehr schnell Wasser auf. Wenn die relative Luftfeuchtigkeit auf 0 % verringert wird, reduziert sich der Wassergehalt der Probe auf 3.1 Gew.-%. Der Wassergehalt bei 50 % relativer Luftfeuchtigkeit am Ende des Meßzykluses liegt bei 7.6 % und das aufgenommene Raman-Spektrum entspricht dem Raman-Spektrum der kristallinen Form V.

### Beispiel 10: Feuchtelagerung der kristallinen Formen II und III

### Lagerbedingungen: 25°C und 60 % relative Luftfeuchtigkeit für 5 Std, 24 Std und 7 Tage

### Versuchsbedingungen:

1.Versuchsreihe:
   Die Substanzen wurden direkt in DSC- bzw TGA-Tiegel eingewogen und diese Tiegel wurden in einer Klimakammer gelagert.
2.Versuchsreihe:
   Je 3 mal 50 mg der Substanzen wurden in 1 mL Vials eingewogen und diese Vials wurden dann offen in einer Klimakammer gelagert.

Die beiden kristallinen Formen II und III zeigen ein unterschiedliches Wasserabsorptionsverhalten. Die Modifikation II nimmt in beiden Versuchen langsamer Wasser auf als die Modifikation III. Während die Modifikation III bereits nach 5 Stunden in der ersten Versuchsreihe 6.70 % bzw. in der zweiten Versuchsreihe 1.92 % Wasser aufgenommen hat, ist bei der Modifikation II keine nennenswerte Wasseraufnahme festzustellen (0.04 % bzw. 0.12 %).

Nach 24 Stunden hat sich bei der Modifikation III in der ersten Versuchsreihe der Wasserwert nicht weiter erhöht (6.28 %), während sich in der zweiten Versuchsreihe nach 24 Stunden einen Anstieg des Wassergehalts auf 6.08 % eingestellt hat. Die Modifikation II hingegen hat nach 24 Stunden in der ersten Versuchsreihe 3.28 % und in der zweiten Versuchsreihe 6.08 % Wasser aufgenommen.

Nach einer Woche Lagerung bei 60 % relativer Luftfeuchtigkeit kann man bei der Modifikation III sowohl in der ersten Versuchsreihe, als auch bei der zweiten Versuchsreihe keine signifikante Wasseraufnahme mehr verzeichnen (Wassergehalt: 6.74 % bzw. 6.83 %). Bei der Modifikation II stellt sich nach einer Woche in beiden Versuchsreihen ein Wassergehalt von 7.03 % bzw. 7.04 % ein.

### Beispiel 11:

### Beispiel 11.1: Bildung Ethanol Solvat

67.1 mg des gemäß Beispiel 1 b hergestellten Hydrochlorids werden in 0.25 mL Ethanol bei 25°C einen Tag lang in Suspension gerührt. Nach Raman und TG-FTIR Analytik liegt ein Ethanol Solvat vor.

Der mittels TG-FTIR bestimmte Masseverlust betrug 8.9 %, wobei Ethanol und wenig Wasser detektiert wurden.

### Beispiel 11.2: Bildung Ethanol Solvat

99.7 mg des gemäß Beispiel 1 b hergestellten Hydrochlorids werden in 0.2 mL Ethanol bei 25°C einen Tag lang in Suspension gerührt. Nach Raman Analytik liegt ein Ethanol Solvat vor.

Aufbewahrung des so erhaltenen Solvates bei Raumtemperatur im Vakuum über Nacht führt nicht zu einer Desolvatisierung. Weitere Aufbewahrung der Probe für 2 Monate in Anwesenheit einer gesättigten Mg(NO₃)₂-Lösung und anschließende Aufbewahrung für 2 Monate in Anwesenheit einer gesättigten NaCl-Lösung führten zum Hydrat Form V.

### Beispiel 11.3: Bildung Aceton Solvat

100 mg des gemäß Beispiel 1 b hergestellten Hydrochlorids wurden für 23.5 Stunden bei 155°C getempert. Wie Raman Analytik zeigt entstand Form II.

51 mg des so erhaltene Materials wurde in 0.1 mL eines Aceton/Wasser(95:5 vol/vol) Gemisches bei 25°C für 2 Tage suspendiert. Raman Analytik zeigte die Bildung einer solvatisierten Form. Der mittels TG-FTIR bestimmte Masseverlust betrug 9.4 %, wobei Aceton und wenig Wasser detektiert wurden.

### Geräte, Methoden:

### Differential Scanning Calorimetrie (DSC):

Gerätebezeichnung Perkin Elmer DSC 7 oder Perkin Eimer Pyris 1. Variable Messungen (Heizungsrate) in Gold-oder Aluminiumtiegeln.

Mettler-Toledo DSC 821, gelochter 40 µl Aluminiumstandardtiegel,variabler Temperaturbereich und variable Heizrate, Stickstoffatmosphäre.

Sofern nicht anders angegeben wurden Substanzmengen im Bereich von 2 bis 20 mg eingesetzt.

### Pulver Röntgenstrahlung Beugungsbilder (PXRD):

PXRD wird mit einem Philips 1710 Pulver X-Strahlung Diffraktometer oder einem Phillips X'Pert PW 3040 durchgeführt, wobei CuK₁₂-Strahlung verwendet wird. D-Abstände werden von den 2θ Werten berechnet, wobei die Wellenlänge von 1.54060 Å zu Grunde gelegt ist. Es gilt allgemein, dass die 2θ Werte eine Fehlerrate von ±0.1-0.2° besitzen. Der experimentelle Fehler bei den d-Abstandswerten ist daher abhängig vom Ort der Linie (des Peaks).

### Raman Spektroskopie:

FT-Raman Spektren werden mit einem Bruker RFS 100 FT-Raman System aufgenommen, der mit einem Nd:YAG Laser (Wellenlänge 1064 nm) und einem mit flüssigem Stickstoff gekühltem Germanium Detektor betrieben wird. Für jede Probe werden 64 Abtastungen mit einer Auflösung von 2 cm⁻¹ akkumuliert. Generell wird eine Laserleistung von 100 mW verwendet.

### TG-FTIR

Netsch Thermo-Microbalance TG209 mit Bruke FT-IR Spektrometer Vektor 22.

Die messungen wurden in Aluminium Tiegel (offen oder mit Mikroloch) unter Stickstoff Atmosphäre durchgeführt. Die Heizrate betrug 10 K/Minute in einem Bereich von 25 - 250°C.

### Beschreibung der Figuren

Figur 1 zeigt das Röntgen-Beugungsbild der polymorphen Form I
Figur 2 zeigt das Raman-Spektrum der polymorphen Form I
Figur 3 zeigt das Röntgen-Beugungsbild der polymorphen Form II
Figur 4 zeigt das Raman-Spektrum der polymorphen Form II
Figur 5 zeigt das Röntgen-Beugungsbild der polymorphen Form III
Figur 6 zeigt das Raman-Spektrum der polymorphen Form III
Figur 7 zeigt das Röntgen-Beugungsbild der polymorphen Form IV
Figur 8 zeigt das Raman-Spektrum der polymorphen Form IV
Figur 9 zeigt das Röntgen-Beugungsbild der polymorphen Form V
Figur 10 zeigt das Raman-Spektrum der polymorphen Form V

Erfindungsgemäße Ausführungsformen (Ausf) sind auch:
Ausf 1. Kristalline Salze von Chlorwasserstoff und 3-[2-(Dimethylamino)methyl-(cyclohex-1-yl)]phenol, bevorzugt 3-[2-Dimethylamino)methyl-(cyclohex-1-yl)-phenol Hydrochlorid der Formel (1) 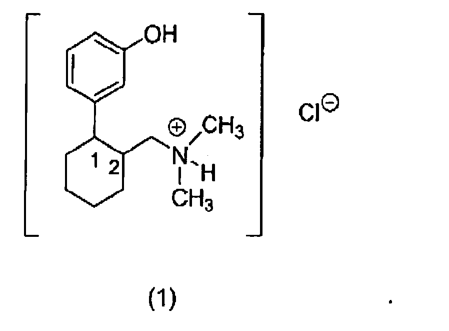
Ausf 2. Salze gemäß Ausf 1, dadurch gekennzeichnet, dass die Verbindung der Formel (1) als Diastereomer oder Gemisch von enantiomeren Diastereomeren mit trans-Konfiguration des Phenylringes und der Dimethylaminomethylgruppe (1R,2R-beziehungsweise 1 S,2S-Konfiguration) vorliegt, bevorzugt als Enantiomer mit der absoluten Konfiguration (1 R,2R) vorliegt.
Ausf 3. Kristalline Form I von 3-[2-Dimethylamino)methy)-(cyclohex-1-yl)-phenol Hydrochlorid der Formel (1) gemäß Ausf 1 oder 2, die ein charakteristisches Röntgen-Beugungsbild im Bereich von 2° bis 35° 2θ mit ausgeprägten charakteristischen Linien aufweist, ausgedrückt in 2 Theta-Werten: 11.2 (w), 14.1 (m), 17.1 (w), 19.5 (w), 19.8 (vs), 20.5 (w), 21.5 (m), 24.1 (m), 26.1 (s), 26.8 (w), 31.3 (m).
Ausf 4. Kristalline Form I von 3-[2-Dimethylamino)methyl-(cyclohex-1-yl)-phenol Hydrochlorid der Formel (1) gemäß Ausf 1 oder 2, die ein Röntgenbeugungsbild wie in Figur 1 dargestellt aufweist.
Ausf 5. Kristalline Form I von 3-[2-Dimethylamino)methyl-(cyclohex-1-yl)-phenol Hydrochlorid der Formel (1) gemäß Ausf 1 oder 2, die ein Raman-Spektrum wie in Figur 2 dargestellt aufweist.
Ausf 6. Kristalline Form II von 3-[2-Dimethylamino)methyl-(cyclohex-1-yl)-phenol Hydrochlorid der Formel (1) gemäß Ausf 1 oder 2, die ein charakteristisches Röntgen-Beugungsbild im Bereich von 2° bis 35° 2θ mit ausgeprägten charakteristischen Linien aufweist, ausgedrückt in 2 Theta-Werten: 11.1 (m), 12.9 (w), 16.1 (m), 17.1 (w), 19.1 (s), 19.6 (w), 19.9 (m), 23.2 (w), 25.8 (w), 26.1 (s), 33.6 (w).
Ausf 7. Kristalline Form II von 3-[2-Dimethylamino)methyl-(cyclohex-1-yl)-phenol Hydrochlorid der Formel (1) gemäß Ausf 1 oder 2, die ein Röntgen-Beugungsbild wie in Figur 3 dargestellt aufweist.
Ausf 8. Kristalline Form II von 3-[2-Dimethylamino)methyl-(cyclohex-1-yl)-phenol Hydrochlorid der Formel (1) gemäß Ausf 1 oder 2, die ein Raman-Spektrum wie in Figur 4 dargestellt aufweist.
Ausf 9. Kristalline Form III von 3-[2-Dimethylamino)methyl-(cyclohex-1-yl)-phenol Hydrochlorid der Formel (1) gemäß Ausf 1 oder 2, die ein charakteristisches Röntgen-Beugungsbild im Bereich von 2° bis 35° 2θ mit ausgeprägten charakteristischen Linien aufweist, ausgedrückt in 2 Theta-Werten: 6.9 (s), 13.9 (m), 16.3 (m), 17.7 (w), 20.9 (vs), 22.1 (w), 22.5 (w), 27.8 (w).
Ausf 10. Kristalline Form III von 3-[2-Dimethylamino)methyl-(cyclohex-1-yl)-phenol Hydrochlorid der Formel (1) gemäß Ausf 1 oder 2, die ein Röntgen-Beugungsbild wie in Figur 5 dargestellt aufweist.
Ausf 11. Kristalline Form III von 3-[2-Dimethylamino)methyl-(cyclohex-1-yl)-phenol Hydrochlorid der Formel (1) gemäß Ausf 1 oder 2, die ein Raman-Spektrum wie in Figur 6 dargestellt aufweist.
Ausf 12. Kristalline Form IV von 3-[2-Dimethylamino)methyl-(cyclohex-1-yl)-phenol Hydrochlorid der Formel (1) gemäß Ausf 1 oder 2, die ein charakteristisches Röntgen-Beugungsbild im Bereich von 2° bis 35° 2θ mit ausgeprägten charakteristischen Linien aufweist, ausgedrückt in 2 Theta-Werten: 12.0 (m), 13.0 (m), 17.3 (m), 17.7 (m), 19.2 (s), 19.7 (m), 20.2 (m), 21.3 (m), 23.4 (m), 24.2 (m), 24.6 (m), 43.1 (vs), 44.2 (vs).
Ausf 13. Kristalline Form IV von 3-[2-Dimethylamino)methyl-(cyclohex-1-yl)-phenol Hydrochlorid der Formel (1) gemäß Ausf 1 oder 2, die ein Röntgen-Beugungsbild wie in Figur 7 dargestellt aufweist.
Ausf 14. Kristalline Form IV von 3-[2-Dimethylamino)methyl-(cyclohex-1-yl)-phenol Hydrochlorid der Formel (1) gemäß Ausf 1 oder 2, die ein Raman-Spektrum wie in Figur 8 dargestellt aufweist.
Ausf 15. Hydrate von 3-[2-Dimethylamino)methyl-(cyclohex-1-yl)-phenol Hydrochlorid der Formel (1) gemäß Ausf 1 oder 2, die einen Anteil an Kristallwasser im Bereich von 1 % bis 10 % bezogen auf das Gewicht des Hydrates aufweisen.
Ausf 16. Hydrate gemäß Ausf 15, dadurch gekennzeichnet, dass der Anteil an Kristallwasser im Bereich von 5 % bis 9 %, bevorzugt von 6 bis 8.5 %, besonders bevorzugt von 7 % bis 8 % liegt.
Ausf 17. Hydrate von 3-[2-Dimethylamino)methyl-(cyclohex-1-yl)-phenol Hydrochlorid der Formel (1) gemäß Ausf 1 oder 2, die ein charakteristisches Röntgen-Beugungsbild im Bereich von 2° bis 35° 2θ mit ausgeprägten charakteristischen Linien aufweist, ausgedrückt in 2 Theta-Werten:
   11.4 (m), 12.1 (m), 16.7 (w), 19.2 (m), 19.4 (w), 20.1 (m), 21.1 (m), 22.4 (vs), 24.0 (m), 31.3 (w);
   nachfolgend als Form V bezeichnet.
Ausf 18. Kristalline Form V von 3-[2-Dimethylamino)methyl-(cyclohex-1-yl)-phenol Hydrochlorid der Formel (1) gemäß Ausf 1 oder 2, die ein Röntgenbeugungsbild wie in Figur 9 dargestellt aufweist.
Ausf 19. Kristalline Form V von 3-[2-Dimethylamino)methyl-(cyclohex-1-yl)-phenol Hydrochlorid der Formel (1) gemäß Ausf 1 oder 2, die ein Raman-Spektrum wie in Figur 10 dargestellt aufweist.
Ausf 20. Verfahren zur Herstellung der kristallinen Form I von 3-[2-Dimethylamino)methyl-(cyclohex-1-yl)-phenol Hydrochlorid gemäß einem oder mehreren der Ausf 3 bis 5, dadurch gekennzeichnet, dass
   a) 3-[2-Dimethylamino)methyl-(cyclohex-1-yl)-phenol Hydrochlorid in der kristallinen Form III und 3-[2-Dimethylamino)methyl-(cyclohex-1-yl)-phenol Hydrochlorid in der kristallinen Form IV oder 3-[2-Dimethylamino)methyl-(cyclohex-1-yl)-phenol Hydrochlorid in der kristallinen Form V in einem Lösungsmittel bis zur vollständigen Bildung der kristallinen Form I gerührt werden, oder
   b) 3-[2-Dimethylamino)methyl-(cyclohex-1-yl)-phenol Hydrochlorid in der kristallinen Form II und phenol Hydrochlorid in der kristallinen Form I in einem Lösungsmittel bis zur vollständigen Bildung der kristallinen Form I gerührt werden.
Ausf 21. Kristalline Form I von 3-[2-Dimethylamino)methyl-(cyclohex-1-yl)-phenol Hydrochlorid der Formel (1) gemäß Ausf 1 oder 2 erhältlich nach einem Verfahren gemäß Ausf 20.
Ausf 22. Verfahren zur Herstellung der kristallinen Form II von 3-[2-Dimethylamino)methyl-(cyclohex-1-yl)-phenol Hydrochlorid gemäß einem oder mehreren der Ausf 6 bis 8, dadurch gekennzeichnet, dass
   a) 3-[2-Dimethylamino)methyl-(cyclohex-1-yl)-phenol Hydrochlorid in der kristallinen Form IV oder eine Mischung von 3-[2-Dimethylamino)methyl-(cyclohex-1-yl)-phenol Hydrochlorid in der kristallinen Form III und 3-[2-Dimethylamino)methyl-(cyclohex-1-yl)-phenol Hydrochlorid in der kristallinen Form V bei einer Temperatur zwischen 150°C und 160°C, bevorzugt bei einer Temperatur zwischen 154°C und 158 °C, bis zur vollständigen Bildung der kristallinen Form II getempert wird, oder
   b) 3-[2-Dimethylamino)methyl-(cyclohex-1-yl)-phenol Hydrochlorid in der kristallinen Form II und 3-[2-Dimethylamino)methyl-(cyclohex-1-yl)-phenol Hydrochlorid in der kristallinen Form III oder 3-[2-Dimethylamino)methyl-(cyclohex-1-yl)-phenol Hydrochlorid in der kristallinen Form IV in einem Lösungsmittel bis zur vollständigen Bildung der kristallinen Form II gerührt wird, oder
   c) eine Suspension der amorphen Form von 3-[2-Dimethylamino)methyl-(cyclohex-1-yl)-phenol Hydrochlorid in einem Lösungsmittel; bevorzugt in einem Lösungsmittel, das keine Solvate bildet; als Träger bei einer Temperatur zwischen 30°C und 50 °C, bevorzugt bei einer Temperatur zwischen 35°C und 45°C, besonders bevorzugt bei einer Temperatur von 40°C, bis zur vollständigen Bildung der kristallinen Form II gerührt wird; oder
   d) 3-[2-Dimethylamino)methyl-(cyclohex-1-yl)-phenol Hydrochlorid in der kristallinen Form III und 3-[2-Dimethylamino)methyl-(cyclohex-1-yl)-phenol Hydrochlorid in der kristallinen Form IV in einem Lösungsmittel bis zur vollständigen Bildung der kristallinen Form II gerührt wird.
Ausf 23. Kristalline Form II von 3-[2-Dimethylamino)methyl-(cyclohex-1-yl)-phenol Hydrochlorid der Formel (1) gemäß Ausf 1 oder 2 erhältlich nach einem Verfahren gemäß Ausf 22.
Ausf 24. Verfahren zur Herstellung der kristallinen Form III von 3-[2-Dimethylamino)methyl-(cyclohex-1-yl)-phenol Hydrochlorid gemäß einem oder mehreren der Ausf 9 bis 11, dadurch gekennzeichnet, dass
   a) 3-[2-Dimethylamino)methyl-(cyclohex-1-yl)-phenol Hydrochlorid in Form eines Ethanol- oder Aceton-Solvates in einem Lösungsmittel gelöst und gerührt und anschließend ausgefällt wird, oder
   b) eine Suspension der amorphen Form von 3-[2-Dimethylamino)methyl-(cyclohex-1-yl)-phenol Hydrochlorid in einem Lösungsmittel; bevorzugt in einem Lösungsmittel, das keine Solvate bildet; als Träger bei einer Temperatur zwischen 30°C und 80°C, bevorzugt bei einer Temperatur zwischen 35°C und 50°C, besonders bevorzugt bei einer Temperatur von 40 °C, bis zur vollständigen Bildung der kristallinen Form III gerührt wird.
Ausf 25. Kristalline Form III von 3-[2-Dimethylamino)methyl-(cyclohex-1-yl)-phenol Hydrochlorid der Formel (1) gemäß Ausf 1 oder 2 erhältlich nach einem Verfahren gemäß Ausf 24.
Ausf 26. Verfahren zur Herstellung der kristallinen Form IV von 3-[2-Dimethylamino)methyl-(cyclohex-1-yl)-phenol Hydrochlorid gemäß einem oder mehreren der Ausf 12 bis 14, dadurch gekennzeichnet, dass
   a) 3-[2-Dimethylamino)methyl-(cyclohex-1-yl)-phenol Hydrochlorid in der kristallinen Form III bei einer Temperatur zwischen 150°C und 160°C, bevorzugt bei einer Temperatur zwischen 154°C und 158°C, bis zur vollständigen Bildung der kristallinen Form IV getempert wird, oder
   b) eine Suspension der amorphen Form von 3-[2-Dimethylamino)methyl-(cyclohex-1-yl)-phenol Hydrochlorid in einem Lösungsmittel; bevorzugt in einem Lösungsmittel, das keine Solvate bildet; als Träger bei einer Temperatur zwischen 40°C und 120°C, bevorzugt bei einer Temperatur zwischen 40°C und 100°C, besonders bevorzugt bei einer Temperatur zwischen 40°C und 80°C, bis zur vollständigen Bildung der kristallinen Form IV gerührt wird; oder
   c) 3-[2-Dimethylamino)methyl-(cyclohex-1-yl)-phenol Hydrochlorid in der kristallinen Form IV und 3-[2-Dimethylamino)methyl-(cyclohex-1-yl)-phenol Hydrochlorid in der kristallinen Form III in einem Lösungsmittel bis zur vollständigen Bildung der kristallinen Form IV gerührt wird.
Ausf 27. Kristalline Form IV von 3-[2-Dimethylamino)methyl-(cyclohex-1-yl)-phenol Hydrochlorid der Formel (1) gemäß Ausf 1 oder 2 erhältlich nach einem Verfahren gemäß Ausf 26.
Ausf 28. Verfahren zur Herstellung der kristallinen Form V von 3-[2-Dimethylamino)methyl-(cyclohex-1-yl)-phenol Hydrochlorid gemäß einem oder mehreren der Ausf 15 bis 19, dadurch gekennzeichnet, dass
   a) 3-[2-Dimethylamino)methyl-(cyclohex-1-yl)-phenol Hydrochlorid in der kristallinen Form I, II, III oder IV an Luft stehen gelassen oder mit Wasserdampf behandelt wird, oder
   b) eine Suspension der amorphen Form von 3-[2-Dimethylamino)methyl-(cyclohex-1-yl)-phenol Hydrochlorid in einer Mischung aus Wasser und ggf. wenigstens einem Lösungsmittel als Träger bel einer Temperatur zwischen 20°C und 60 °C, bevorzugt bei einer Temperatur zwischen 20°C und 30°C, gerührt wird und anschließend das verbleibende Wasser bzw. Lösungsmittel entfernt wird.
Ausf 29. Kristalline Form V von 3-[2-Dimethylamino)methyl-(cyclohex-1-yl)-phenol Hydrochlorid der Formel (1) gemäß Ausf 1 oder 2 erhältlich nach einem Verfahren gemäß Ausf 28.
Ausf 30. Pharmazeutische Zusammensetzung, enthaltend eine wirksame Menge 3-[2-Dimethylamino)methyl-(cyclohex-1-yl)-phenol Hydrochlorid der Formel (1) gemäß einem oder mehreren der Ausf 1 bis 19, 21, 23, 25, 27 und 29 und einen pharmazeutischen Träger oder ein pharmazeutisches Verdünnungsmittel.
Ausf 31. Zusammensetzung gemäß Ausf 30, worin die Verbindung der Formel (1) als kristalline Form I, kristalline Form II, kristalline Form III, kristalline Form IV, kristalline Form V oder in einer Mischung der Formen I, II, III, IV und V vorliegt.
Ausf 32. Zusammensetzung gemäß Ausf 31, worin die Verbindung der Formel (1) als kristalline Form II enthalten ist.
Ausf 33. Zusammensetzung gemäß Ausf 31, worin die Verbindung der Formel (1) als kristalline Form V enthalten ist.
Ausf 34. Verwendung von 3-[2-Dimethylamino)methyl-(cyclohex-1-yl)-phenol Hydrochlorid der Formel (1) gemäß einem oder mehreren der Ausf 1 bis 19, 21, 23, 25, 27 und 29 zur Herstellung einer pharmazeutischen Zusammensetzung, insbesondere für die Behandlung von Schmerzzuständen.
Ausf 35. Verwendung von 3-[2-Dimethylamino)methyl-(cyclohex-1-yl)-phenol Hydrochlorid der Formel (1) gemäß einem oder mehreren der Ausf 1 bis 19, 21, 23, 25, 27 und 29 als Wirkstoff in Arzneimitteln, bevorzugt als Wirkstoff in Schmerzmitteln.
Ausf 36. Verfahren zur Behandlung von Schmerzzuständen, bei dem man einem unter Schmerzen leidenden Patienten eine wirksame Menge von 3-[2-Dimethylamino)methyl-(cyclohex-1-yl)-phenol Hydrochlorid der Formel (1) gemäß einem oder mehreren der Ausf 1 bis 19, 21, 23, 25, 27 und 29 verabreicht.

## Patentansprüche

1. Kristalline Salze von Chlorwasserstoff und 3-[2-(Dimethylamino)methyl-(cyclohex-1-yl)]phenol, bevorzugt 3-[2-Dimethylamino)methyl-(cyclohex-1-yl)-phenol Hydrochlorid der Formel (1)

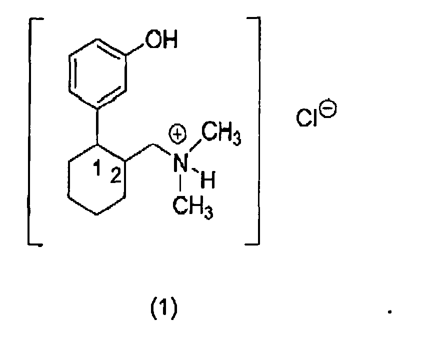

2. Salze gemäß Anspruch 1, **dadurch gekennzeichnet, dass** die Verbindung der Formel (1) als Diastereomer oder Gemisch von enantiomeren Diastereomeren mit trans-Konfiguration des Phenylringes und der Dimethylaminomethylgruppe (1 R,2R- beziehungsweise 1S,2S-Konfiguration) vorliegt, bevorzugt als Enantiomer mit der absoluten Konfiguration (1 R,2R) vorliegt.

3. Kristalline Form I von 3-[2-Dimethylamino)methyl-(cyclohex-1-yl)-phenol Hydrochlorid der Formel (1) gemäß Anspruch 1 oder 2, die ein charakteristisches Röntgen-Beugungsbild im Bereich von 2° bis 35° 2θ mit ausgeprägten charakteristischen Linien aufweist, ausgedrückt in 2 Theta-Werten:
11.2 (w), 14.1 (m), 17.1 (w), 19.5 (w), 19.8 (vs), 20.5 (w), 21.5 (m), 24.1 (m), 26.1 (s), 26.8 (w), 31.3 (m).

4. Kristalline Form I von 3-[2-Dimethylamino)methyl-(cyclohex-1-yl)-phenol Hydrochlorid der Formel (1) gemäß Anspruch 1 oder 2, die ein Röntgenbeugungsbild wie in Figur 1 dargestellt aufweist.

5. Kristalline Form I von 3-[2-Dimethylamino)methyl-(cyclohex-1-yl)-phenol Hydrochlorid der Formel (1) gemäß Anspruch 1 oder 2, die ein Raman-Spektrum wie in Figur 2 dargestellt aufweist.

6. Kristalline Form III von 3-[2-Dimethylamino)methyl-(cyclohex-1-yl)-phenol Hydrochlorid der Formel (1) gemäß Anspruch 1 oder 2, die ein charakteristisches Röntgen-Beugungsbild im Bereich von 2° bis 35° 2θ mit ausgeprägten charakteristischen Linien aufweist, ausgedrückt in 2 Theta-Werten:
6.9 (s), 13.9 (m), 16.3 (m), 17.7 (w), 20.9 (vs), 22.1 (w), 22.5 (w), 27.8 (w).

7. Kristalline Form III von 3-[2-Dimethylamino)methyl-(cyclohex-1-yl)-phenol Hydrochlorid der Formel (1) gemäß Anspruch 1 oder 2, die ein Röntgen-Beugungsbild wie in Figur 5 dargestellt aufweist.

8. Kristalline Form III von 3-[2-Dimethylamino)methyl-(cyclohex-1-yl)-phenol Hydrochlorid der Formel (1) gemäß Anspruch 1 oder 2, die ein Raman-Spektrum wie in Figur 6 dargestellt aufweist.

9. Kristalline Form IV von 3-[2-Dimethylamino)methyl-(cyclohex-1-yl)-phenol Hydrochlorid der Formel (1) gemäß Anspruch 1 oder 2, die ein charakteristisches Röntgen-Beugungsbild im Bereich von 2° bis 35° 2θ mit ausgeprägten charakteristischen Linien aufweist, ausgedrückt in 2 Theta-Werten:
12.0 (m), 13.0 (m), 17.3 (m), 17.7 (m), 19.2 (s), 19.7 (m), 20.2 (m), 21.3 (m), 23.4 (m), 24.2 (m), 24.6 (m), 43.1 (vs), 44.2 (vs).

10. Kristalline Form IV von 3-[2-Dimethylamino)methyl-(cyclohex-1-yl)-phenol Hydrochlorid der Formel (1) gemäß Anspruch 1 oder 2, die ein Röntgen-Beugungsbild wie in Figur 7 dargestellt aufweist.

11. Kristalline Form IV von 3-[2-Dimethylamino)methyl-(cyclohex-1-yl)-phenol Hydrochlorid der Formel (1) gemäß Anspruch 1 oder 2, die ein Raman-Spektrum wie in Figur 8 dargestellt aufweist.

12. Verfahren zur Herstellung der kristallinen Form I von 3-[2-Dimethylamino)methyl-(cyclohex-1-yl)-phenol Hydrochlorid gemäß einem oder mehreren der Ansprüche 3 bis 5, **dadurch gekennzeichnet, dass**
a) 3-[2-Dimethylamino)methyl-(cyclohex-1-yl)-phenol Hydrochlorid in der kristallinen Form III und 3-[2-Dimethylamino)methyl-(cyclohex-1-yl)-phenol Hydrochlorid in der kristallinen Form IV oder 3-[2-Dimethylamino)methyl-(cyclohex-1-yl)-phenol Hydrochlorid in der kristallinen Form V in einem Lösungsmittel bis zur vollständigen Bildung der kristallinen Form I gerührt werden, oder
b) 3-[2-Dimethylamino)methyl-(cyclohex-1-yl)-phenol Hydrochlorid in der kristallinen Form II und 3-[2-Dimethylamino)methyl-(cyclohex-1-yl)-phenol Hydrochlorid in der kristallinen Form **I** in einem Lösungsmittel bis zur vollständigen Bildung der kristallinen Form I gerührt werden.

13. Kristalline Form I von 3-[2-Dimethylamino)methyl-(cyclohex-1-yl)-phenol Hydrochlorid der Formel (1) gemäß Anspruch 1 oder 2 erhältlich nach einem Verfahren gemäß Anspruch 12.

14. Verfahren zur Herstellung der kristallinen Form III von 3-[2-Dimethylamino)methyl-(cyclohex-1-yl)-phenol Hydrochlorid gemäß einem oder mehreren der Ansprüche 6 bis 8, **dadurch gekennzeichnet, dass**
a) 3-[2-Dimethylamino)methyl-(cyclohex-1-yl)-phenol Hydrochlorid in Form eines Ethanol- oder Aceton-Solvates in einem Lösungsmittel gelöst und gerührt und anschließend ausgefällt wird, oder
b) eine Suspension der amorphen Form von 3-[2-Dimethylamino)methyl-(cyclohex-1-yl)-phenol Hydrochlorid in einem Lösungsmittel; bevorzugt in einem Lösungsmittel, das keine Solvate bildet; als Träger bei einer Temperatur zwischen 30 °C und 80°C, bevorzugt bei einer Temperatur zwischen 35°C und 50°C, besonders bevorzugt bei einer Temperatur von 40°C, bis zur vollständigen Bildung der kristallinen Form III gerührt wird.

15. Kristalline Form III von 3-[2-Dimethylamino)methyl-(cyclohex-1-yl)-phenol Hydrochlorid der Formel (1) gemäß Anspruch 1 oder 2 erhältlich nach einem Verfahren gemäß Anspruch 14.

16. Verfahren zur Herstellung der kristallinen Form IV von 3-[2-Dimethylamino)methyl-(cyclohex-1-yl)-phenol Hydrochlorid gemäß einem oder mehreren der Ansprüche 9 bis 11, **dadurch gekennzeichnet, dass**
a) 3-[2-Dimethylamino)methyl-(cyclohex-1-yl)-phenol Hydrochlorid in der kristallinen Form III bei einer Temperatur zwischen 150°C und 160 °C, bevorzugt bei einer Temperatur zwischen 154°C und 158°C, bis zur vollständigen Bildung der kristallinen Form IV getempert wird, oder
b) eine Suspension der amorphen Form von 3-[2-Dimethylamino)methyl-(cyclohex-1-yl)-phenol Hydrochlorid in einem Lösungsmittel; bevorzugt in einem Lösungsmittel, das keine Solvate bildet; als Träger bei einer Temperatur zwischen 40°C und 120°C, bevorzugt bei einer Temperatur zwischen 40°C und 100 °C, besonders bevorzugt bei einer Temperatur zwischen 40°C und 80°C, bis zur vollständigen Bildung der kristallinen Form IV gerührt wird; oder
c) 3-[2-Dimethylamino)methyl-(cyclohex-1-yl)-phenol Hydrochlorid in der kristallinen Form IV und 3-[2-Dimethylamino)methyl-(cyclohex-1-yl)-phenol Hydrochlorid in der kristallinen Form III in einem Lösungsmittel bis zur vollständigen Bildung der kristallinen Form IV gerührt wird.

17. Kristalline Form IV von 3-[2-Dimethylamino)methyl-(cyclohex-1-yl)-phenol Hydrochlorid der Formel (1) gemäß Anspruch 1 oder 2 erhältlich nach einem Verfahren gemäß Anspruch 16.

18. Pharmazeutische Zusammensetzung, enthaltend eine wirksame Menge 3-[2-Dimethylamino)methyl-(cyclohex-1-yl)-phenol Hydrochlorid der Formel (1) gemäß einem oder mehreren der Ansprüche 1-5, 6-8, 9-11, 13, 15 und 17 und einen pharmazeutischen Träger oder ein pharmazeutisches Verdünnungsmittel.

19. Zusammensetzung gemäß Anspruch 18, worin die Verbindung der Formel (1) als kristalline Form I, kristalline Form III oder kristalline Form IV oder In einer Mischung der Formen I, III und IV vorliegt.

20. Verwendung von 3-[2-Dimethylamino)methyl-(cyclohex-1-yl)-phenol Hydrochlorid der Formel (1) gemäß einem oder mehreren der Ansprüche 1-5, 6-8, 9-11, 13, 75 und 17 zur Herstellung einer pharmazeutischen Zusammensetzung, insbesondere für die Behandlung von Schmerzzuständen.

21. Verwendung von 3-[2-Dimethylamino)methyl-(cyclohex-1-yl)-phenol Hydrochlorid der Formel (1) gemäß einem oder mehreren der Ansprüche 1-5, 6-8, 9-11, 13, 15 und 17 als Wirkstoff in Arzneimitteln, bevorzugt als Wirkstoff in Schmerzmitteln.

22. Verfahren zur Behandlung von Schmerzzuständen, bei dem man einem unter Schmerzen leidenden Patienten eine wirksame Menge von 3-[2-Dimethylamino)methyl-(cyclohex-1-yl)-phenol Hydrochlorid der Formel (1) gemäß einem oder mehreren der Ansprüche 1-5, 6-8, 9-11, 13, 15 und 17 verabreicht.
